# EUROPEAN PATENT SPECIFICATION

(11) **EP 2 769 672 B1**
(45) Date of publication and mention of the grant of the patent: **11.11.2020**
(21) Application number: 14156031.8
(22) Date of filing: 20.02.2014
(51) Int. Cl.: A61B 5/055, A61B 5/1455, A61B 5/1459, A61B 5/026

(54) **System for in vivo and magnetic resonance investigation of kidney function**
System zur In-vivo- und Magnetresonanzuntersuchung der Nierenfunktion
Système d'examen par résonance magnétique et in vivo de la fonction rénale

(30) Priority: 20.02.2013 EP 13156027; 21.02.2013 EP 13156151; 21.02.2013 EP 13156162
(43) Date of publication of application: 27.08.2014
(73) Proprietor: Max-Delbrück-Centrum für Molekulare Medizin, 13125 Berlin (DE)
(72) Inventor: Arakelyan, Karen, 14052 Berlin (DE); Cantow, Kathleen, 10409 Berlin (DE); Flemming, Bert, 10318 Berlin (DE); Hentschel, Jan, 10249 Berlin (DE); Niendorf, Thoralf, 52074 Aachen (DE); Pohlmann, Andreas, 16321 Bernau (DE); Seeliger, Erdmann, 13187 Berlin (DE); Ladwig, Mechthild, 13581 Berlin (DE)
(74) Representative: Hertin und Partner Rechts- und Patentanwälte PartG mbB

(56) References cited:
- ELISABETE ALCANTARA DOS SANTOS ET AL: "Early Changes With Diabetes in Renal Medullary Hemodynamics as Evaluated by Fiberoptic Probes and BOLD Magnetic Resonance Imaging", NIH Public Access Author Manuscript , 2007, pages 1-13, XP055119666, Retrieved from the Internet: URL:http://www.ncbi.nlm.nih.gov/pmc/articl es/PMC2904752/pdf/nihms157871.pdf [retrieved on 2014-05-23]
- "22nd ESICM Annual Congress, Posters_Tuesday 13 October 2009", INTENSIVE CARE MEDICINE, vol. 35, no. 1, 6 August 2009 (2009-08-06) , pages 120-204, XP019735637, SPRINGER, BERLIN, DE ISSN: 1432-1238, DOI: 10.1007/S00134-009-1596-Z
- LAURENT JUILLARD ET AL: "Blood oxygen level-dependent measurement of acute intra-renal ischemia", KIDNEY INTERNATIONAL, vol. 65, no. 3, 1 March 2004 (2004-03-01), pages 944-950, XP055119741, ISSN: 0085-2538, DOI: 10.1111/j.1523-1755.2004.00469.x
- Anonymous: "Precision Perivascular Flowprobes", transonic , December 2012 (2012-12), page 6PP, XP002724866, Retrieved from the Internet: URL:http://www.transonic.com/data/RL-26-fl y.pdf [retrieved on 2014-05-23]
- Tomaz Jann ET AL: "EFFECT OF HYDRALAZINE ON BLOOD FLOW, OXYGENATION, AND INTERSTITIAL FLUID PRESSURE IN SUBCUTANEOUS TUMORS", , 1 January 2003 (2003-01-01), XP055119678, Retrieved from the Internet: URL:http://lbk.fe.uni-lj.si/pdfs/aem2003a. pdf [retrieved on 2014-05-22]
- Anonymous: "RF Coils", Bruker BioSpin , 26 November 2011 (2011-11-26), XP002724867, Retrieved from the Internet: URL:https://web.archive.org/web/2011112610 3823/http://www.bruker-biospin.com/rf_coil s.html [retrieved on 2014-05-22]
- Anonymous: "BioSpec MR Imaging and in vivo Spectroscopy at High Magnetic FIelds", Bruker , 1 March 2005 (2005-03-01), pages 1-20, XP055119652, Retrieved from the Internet: URL:http://weill.cornell.edu/cbic/faciliti es/biospec_apps.pdf [retrieved on 2014-05-22]
- A. POHLMANN ET AL: "Linking non-invasive parametric MRI with invasive physiological measurements (MR-PHYSIOL): towards a hybrid and integrated approach for investigation of acute kidney injury in rats", ACTA PHYSIOLOGICA, vol. 207, no. 4, 20 February 2013 (2013-02-20), pages 673-689, XP055119537, ISSN: 1748-1708, DOI: 10.1111/apha.12065
- T. WHITEHOUSE: "Tissue oxygen and hemodynamics in renal medulla, cortex, and corticomedullary junction during hemorrhage-reperfusion", AJP: RENAL PHYSIOLOGY, vol. 291, no. 3, 4 April 2006 (2006-04-04) , pages F647-F653, XP055119610, ISSN: 0363-6127, DOI: 10.1152/ajprenal.00475.2005
- Anonymous: "Precision Perivascular Flowprobes TS420 Modules", , 11 October 2011 (2011-10-11), pages 1-6, XP055607709, Retrieved from the Internet: URL:https://web.archive.org/web/2011101117 1904/http://www.transonic.com/data/RL-26-f ly.pdf [retrieved on 2019-07-22]

## Description

The present invention relates to the technical fields of invasive and non-invasive analytical technology for assessing biological organ structure and function. The invention provides a measurement system and device for simultaneous determination of physiological parameters of a kidney by in vivo and magnetic resonance imaging (MRI) techniques, comprising one or more probes for measuring in vivo physiological parameters, an animal holding device and an MRI-scanner.

In one embodiment the invention therefore relates to a system and device for simultaneous determination of renal perfusion and renal oxygenation via in vivo physiological measurement and magnetic resonance imaging (MRI). The system comprises preferably a perivascular flow probe for measuring renal blood flow, two oxygen probes for measuring cortical and medullary oxygen saturation (partial pressure of oxygen; pO2), respectively, in addition to an animal holding device and an MRI-scanner.

Acute kidney injuries of various origins share a common link in the pathophysiological chain of events: imbalance between renal medullary oxygen delivery and oxygen demand. For *in vivo* assessment of kidney haemodynamics and oxygenation in animals quantitative but invasive physiological methods are established. A very limited number of studies attempted to link these invasive methods with parametric Magnetic Resonance Imaging (MRI) of the kidney. Moreover, the validity of parametric MRI (pMRI) as a surrogate marker for renal tissue perfusion and renal oxygenation has not been systematically examined yet. The invention therefore provides a combination of in vivo techniques and non-invasive MRI in an integrated hybrid setup with the goal to calibrate, monitor and interpret parametric MR and physiological parameters by means of standardized analysis.

A multi-modal measurement system is provided, capable of providing a technical solution that balances the needs of *in vivo* physiological measurements together with the constraints dictated by small bore MR scanners. As an example a prototype of the integrated approach is demonstrated herein including determination of hypoxia and hyperoxia in a single kidney of an animal model.

### BACKGROUND OF THE INVENTION

Acute kidney injury (AKI) is clinically characterized by the rapid loss of renal excretory function. A variety of causes can trigger AKI including renal ischemic events, cardiac disorders and impaired systemic circulation, hepatic malfunction, sepsis, rhabdomyolysis and following exposure to nephrotoxic substances. Although AKI is potentially reversible, it is one of the leading causes of in-hospital mortality. In spite of potential recovery from AKI, patients remain predisposed to long-term impairment or loss of kidney function (Bellomo et al., 2012, Bonventre and Yang, 2011, Srisawat and Kellum, 2011, Twombley et al., 2011, Yap and Lee, 2012, Zarjou and Agarwal, 2011) AKI pathophysiology remains to be unravelled. Prevention and therapy largely rely on empirical clinical knowledge. Translational approaches - although urgently warranted - remain scarce (Zarjou et al., 2012). A plethora of pathophysiological factors may contribute to the development of AKI. Probably acting in concert in various combinations, factors include oxidative stress, cytotoxic, inflammatory, apoptotic, rheologic, metabolic and vasoactive factors (Aksu et al., 2011, Bonventre and Yang, 2011, Bougle and Duranteau, 2011, Heyman et al., 2012, Heyman et al., 2010, Le Dorze et al., 2009, Seeligeret al., 2012, Sharfuddin and Molitoris, 2011, Singh et al., 2008, Tumlin, 2009, Yap and Lee, 2012). Animal studies suggest that AKI of various origins share one common link in the pathophysiological chain of events, ultimately leading to AKI: imbalance between renal medullary oxygen delivery and oxygen demand (Brezis and Rosen, 1995, Evans et al., 2011a, Flemming et al., 2000, Heyman et al., 2012, Heyman et al., 2008, Hoff et al., 2011, Le Dorze et al., 2009, Legrand et al., 2008, Liss et al., 2009, O'Connor et al., 2006b, Regner and Roman, 2012, Schurek, 1988, Seeliger et al., 2007, Seeliger et al., 2012).

Notwithstanding the success and utility of quantitative *in vivo* methods established for invasive characterization of renal hemodynamics and oxygenation, the particularities of renal perfusion and oxygenation present a challenge for making conclusive diagnostic statements on the pivotal role of renal medullary hypoperfusion and hypoxia in AKI. In order to shed light on to the pathophysiology of AKI, methods providing spatio-temporal coverage of the kidney are required.

### Renal Perfusion and Oxygenation

Oxygen delivery is determined by blood flow and by arterial oxygen content. The major determinant of renal O2 consumption is energy-dependent tubular reabsorption. Renal hemodynamics and oxygenation offer a number of striking differences versus non-renal tissues. Whereas O2 consumption determines perfusion in non-renal tissues, renal O2 consumption is largely determined by perfusion. Increased renal blood flow (RBF) is, in general, accompanied by increased glomerular filtration rate (GFR), and therefore necessitates increased energy-dependent tubular reabsorbtion (Blantz and Deng, 2007, Blantz et al., 2007, Brezis et al., 1994, Evans et al., 2008a, O'Connor, 2006, Rosen et al., 1992, Pitts, 1974). Another particularity is the highly heterogeneous blood flow through the kidney. On a per gram basis whole-kidney blood flow is higher than that of most other tissues. However, blood flow distribution within the kidney differs substantially. Cortical perfusion amounts to about 90% of total RBF, whereas medullary perfusion amounts to only 10%. Even intra-layer (cortex, outer medulla, inner medulla) perfusion is quiet heterogeneous (Edwards et al., 2000, Moffat and Fourman, 1963, Navar et al., 1996, Pitts, 1974). In accordance with total RBF, the arterio-venous difference in O2 content of the whole kidney is small as compared to other tissues. But the partial pressure of oxygen (pO2) is low in the medulla and varies within the respective layer, which is related to the non-uniform perfusion (Baumgartl et al., 1972, Blantz et al., 2007, Blantz and Weir, 2004, Evans et al., 2008a, Lubbers and Baumgartl, 1997, O'Connor, 2006, O'Connor et al., 2006a, O'Connor et al., 2006b, Seeliger et al., 2007, Welch et al., 2001, Leichtweiss et al., 1969). However, three other effects substantially contribute to low regional pO2. First, the particular architecture of intra-renal vasculature enables arterio-venous O2 shunt diffusion both in the medulla and the cortex (Evans et al., 2008a, Gardiner et al., 2012, Leong et al., 2007, O'Connor, 2006, O'Connor et al., 2006a, O'Connor et al., 2006b, Schurek et al., 1990, Dantzler et al., 2011). Second, the hematocrit in the long and narrow vasa recta supplying the medulla and, thus, the O2 content of blood perfusing this layer is low due to the Fåhraeus-Lindquist effect (Edwards et al., 2000, Zimmerhackl et al., 1985). Finally, renal vascular architecture promotes the phenomenon of "plasma skimming", i.e., differential distribution of erythrocytes and plasma at certain vessel branches that results in different hematocrit and O2 content of blood perfusing the daughter vessels (Edwards et al., 2000, Jonsson et al., 1992, Pappenheimer and Kinter, 1956).

The kidney is equipped with efficient mechanisms of autoregulation, i.e., the ability to maintain RBF and GFR relatively constant in the face of moderate changes in renal perfusion pressure (RPP). Renal autoregulatory mechanisms include the myogenic mechanism, the tubulo-glomerular feedback (TGF), and a third mechanism that has been discovered recently (Just, 2007, Seeliger et al., 2009, Wronski et al., 2003). The third mechanism of RBF autoregulation was first detected by studies on the dynamic properties of autoregulation in anesthetized rats, dogs, and mice. These studies used the step-response technique which provides means to distinguish the mechanisms of autoregulation of the intact kidney *in vivo* according to their frequency characteristics. Beside the long-known high frequency myogenic response (0.1 - 0.2 Hz) and the low frequency tubulo-glomerular feed-back (0.02 - 0.04 Hz in rats), a third mechanism was detected that operates at an even lower frequency (< 0.01 Hz) (Just et al., 2001, Just et al., 2002, Wronski et al., 2003). Renal autoregulatory mechanisms, in particular the TGF and the third mechanism, have been suggested to serve the purpose of balancing O2 delivery, i.e. RBF with metabolic and O2 demands arising from tubular reabsorption. It is also conceivable that in the setting of AKI autoregulatory mechanisms may lead to a vicious circle in which low perfusion results in tissue hypoxia that in return further reduces perfusion (Blantz and Weir, 2004, O'Connor, 2006, O'Connor et al., 2006a, O'Connor et al., 2006b, Seeliger et al., 2009, Wronski et al., 2003). The outer medulla is particularly prone to imbalance between O2 delivery and demand since this layer exhibits a high O2 demand but low pO2.

The differential perfusion and oxygenation of renal tissues is subject to changes induced by a variety of physiological and pathophysiological conditions. Redistribution of perfusion and/or of pO2 have been reported to be induced by graded reduction in RPP, by vasoactive factors such as adenosine, nitric oxide, angiotensin II, by hormones like vasopressin, by altered rheological properties of blood, by diuretics, by x-ray contrast media, by ischemia/reperfusion injury and by endotoxin shock (Badzynska and Sadowski, 2009, Dobrowolski and Sadowski, 2005, Evans et al., 1998, Evans et al., 2010, Evans et al., 2011b, Evans et al., 2000, Flemming et al., 2000, Jensen et al., 2009, Navar et al., 1996, Olof et al., 1991, Seeliger et al., 2007, Warner et al., 2009, Ahmeda and Johns, 2012, Badzynska and Sadowski, 2012, Hussein et al., 2012).

### In vivo Characterization of Kidney Hemodynamics and Oxygenation

*In vivo* characterization of kidney hemodynamics and oxygenation comprise invasive methods that assess (i) perfusion and O2-consumption of the entire kidney and (ii) regional perfusion and oxygenation in the intact animal. Today's "gold-standard" for whole kidney blood flow (RBF) measurements is a flow probe (Transonic) attached to the renal artery or to the renal vein that makes use of ultra-sound transit time differences (Dean et al., 1996). This method provides continuous absolute measurements of blood flow (in ml min-1). Whole-kidney O2-consumption can be assessed by means of the renal-arterial to renal-venous difference in O2 content multiplied by RBF. This method requires simultaneous blood sampling from the renal artery and the renal vein. It does not usually enable continuous measurements (Evans et al., 2008b).

Laser-Doppler probes can estimate regional blood perfusion within specific local areas of tissue close to the probe (Edwards et al., 2000). For this purpose, pulsed light of a distinct wave length is guided into the subjacent tissue using a fiber optic probe. From the amount of reflected light - which is fed back to the apparatus via the same fiber optic probe - the concentration of erythrocytes per tissue volume unit is determined. The average velocity of erythrocyte movements is derived from the frequency shift (Doppler effect) between transmitted and reflected light. From both measures, an estimate of blood flow is calculated. Since the signal depends on the actual haematocrit (which is influenced by the effects of plasma skimming), it is not an absolute measurement of blood flow and hence is termed Laser-flux-signal (Edwards et al., 2000, Evans et al., 2008b).

For many decades, Clark-type electrodes have been used to measure local tisssue pO2. In recent years, probes became available that make use of fluorescence quenching induced in crystals of a fluorescent dye attached to the tip of a fibre optic probe. When excited by laser light these crystals fluoresce with the fluorescence being reduced by oxygen ("quenching") (Evans et al., 2008b, Leong et al., 2008). This approach allows precise measurements of absolute pO2 values (in mm Hg) even at very low pO2 common in the renal medulla. The high fidelity is due to two reasons: (i) the inverse relationship between pO2 and signal strength and (ii) the optode does not consume oxygen. The surface of the dye-armed tip is larger (230 Vm diameter) than that of common Clark-type electrodes. Consequently, pO2 of a larger tissue area is integrated, which results in more representative values (Leong et al., 2008). Recently developed combined optical Laser-Doppler-flux and pO2 probes enable simultaneous measurements of flux and pO2 within the same volume of tissue (Baudelet and Gallez, 2002). The Laser-Doppler-flux and the pO2 probe approach share a significant drawback, since data are obtained within a rather small volume of tissue. Considering the perfusion and oxygenation heterogeneity within a given kidney layer, extrapolation of the results to other areas of the same layer must be made with due caution. Notwithstanding its utility in animal experiments, the *in vivo* methods commonly used for quantitative characterization of renal perfusion and oxygenation are invasive. This constraint presents a severe challenge for longitudinal studies. Thus, a non-invasive *in vivo* method is required for longitudinal studies. Heterogeneity of tissue perfusion and oxygenation relies on the kidney-specific vascular architecture and the mutual interactions between perfusion and oxygenation that result in effects like arterio-venous shunt diffusions and plasma skimming. Considering that these effects are perturbed by a variety of physiological and pathophysiological conditions, MRI offers alternative means to obtain insight into the mechanisms that control perfusion and oxygenation under physiological conditions and in kidney disorders such as AKI.

### Assessment of Kidney Hemodynamics and Oxygenation with MRI

MRI provides images of high soft-tissue contrast and (sub)millimeter spatial resolution non-invasively. MRI has evolved rapidly over the past quarter of a century, allowing for applications across a broad spectrum of basic science, clinical research and diagnostic imaging areas. This includes imaging anatomy, function, probing for total water content, perfusion, tissue water diffusion and oxygenation.

Blood-oxygenation level dependent (BOLD) MRI is susceptible to magnetic field perturbations induced by paramagnetic deoxyhaemoglobin which affects the relaxation times T2* and T2 (Ogawa et al., 1990, Silvennoinen et al., 2003, Zhao et al., 2007). T2* relaxation refers to decay of transverse magnetization caused by a combination of spin-spin relaxation (T2) and magnetic susceptibility driven dephasing (T2') which can be described by 1/ T2*=1/ T2*+1/ T2'. T2* is sensitive to the concentration of deoxygenated haemoglobin per tissue volume element (voxel) which depends not only on O2-saturation of haemoglobin, but also on blood vessel volume per tissue volume element, and the concentration of haemoglobin per blood volume unit (i.e., the hematocrit) (Notohamiprodjo et al., 2010). Also, T2* and blood oxygenation induced T2* changes exhibit a linear relationship with the magnetic field strength (Turner et al., 1993, van der Zwaag et al., 2009, Donahue et al., 2011). T2* weighted MRI rather provides a surrogate than a quantitative measure of oxygen tension. It should be also noted that T2* weighted MRI reflects oxygenation of blood rather than tissue (Notohamiprodjo et al., 2010). This distinction is of relevance since renal blood oxygenation and renal tissue oxygenation can vary independently as a result of both arterio-venous O2- shunting and plasma skimming (Evans et al., 2008b, Evans et al., 2007). Changes in [vessel volume]/[tissue volume] induced by active vasomotion or passive circular distension also alter T2* (Evans et al., 2008b). Parametric T2*/T2 mapping using a set of images each encoded with a different T2* or T2 weighting provides quantitative data of the relaxation times T2* and T2. This approach provides means for T2* or T2 monitoring rather than tracking signal intensity changes in individual T2* or T2 weighted images.

T2* weighted MRI, mapping of T2* or its reciprocal value R2* (R2*=1/T2*) as well as T2' mapping have been used in clinical and preclinical settings to examine the physiology of kidney oxygenation (Evans et al., 2008b, Li et al., 2008, Notohamiprodjo et al., 2010, Prasad, 2006, Mathys et al., 2011). Basic and translational research reports eloquently speak about the use of T2* weighted MRI in a broad range of physiological and pathophysiological settings. Various stimuli were used to study regulation of renal perfusion and oxygenation in animals under physiological conditions. For this purpose vasoactive agents like nitric oxide (Li et al., 2003, Li et al., 2009), prostaglandines (Prasad et al., 2001), reductions in RPP (Alford et al., 2005, Juillard et al., 2004) , water diuresis (Epstein et al., 2002, Haque et al., 2011, Prasad and Epstein, 1999), and changes in inspiratory oxygen fraction (Pedersen et al., 2005) were used to modify renal perfusion and oxygen delivery. To lower renal oxygen consumption, diuretics that reduce tubular reabsorption were applied (Gomez et al., 2009, Li et al., 2004, Warner et al., 2011). Animal models of kidney disorders studied by means of T2* weighted MRI include nephropathies due to diabetes (dos Santos et al., 2007, Edlund et al., 2009, Ries et al., 2003), x-ray contrast media (Haneder et al., 2012, Prasad et al., 2001, Zhang et al., 2012), hypertension (Li et al., 2003, Li et al., 2005), ischemia/reperfusion (Oostendorp et al., 2011), and renal artery stenosis (Rognant et al., 2011).

A very limited number of studies attempted to link established invasive methods with T2* weighted renal MRI in order to explore the applicability of MRI for the detection of changes in renal perfusion and oxygenation (dos Santos et al., 2007, Li et al., 2009). For this purpose, the effects of interventions on renal T2* were benchmarked against local tissue pO2 and perfusion by comparing independent cohorts of animals, which underwent either invasive measurements or MRI (dos Santos et al., 2007, Li et al., 2009, Prasad et al., 2001). In one study, T2* changes obtained for the contralateral kidney were compared with local tissue pO2 data simultaneously obtained from the ipsilateral kidney using Clark-electrodes (Pedersen et al., 2005). RBF deduced from Transonic probes was compared with first pass contrast bolus MRI (Ludemann et al., 2009) and with T2* weighted MRI (Juillard et al., 2004) in pigs using large bore whole body MR scanners. Unfortuntaly first pass contrast bolus MRI offers limited suitibility for longitudinal studies due to the time constraints dictated by the kinetics of the contrast agent wash out with blood half live times exceeding the temporal resolution shown here by an order of magnitude.

To summarize, T2*/T2 weighted MRI can provide parametric maps of the MR relaxation parameters T2* and T2, is non-invasive, provides a (sub)minute temporal resolution, supports longitudinal studies and holds the potential to monitor changes in renal perfusion and oxygenation qualitatively.

The validity and efficacy of these techniques for quantitative characterisation of local tissue perfusion and oxygenation and its changes under different functional conditions has not been systematically examined yet.

At present no tools are available for reliable simultaneous in vivo and MRI kidney analysis, thereby leaving the application of MRI on its own for kidney analysis in some state of uncertainty. Novel means for assessing MRI-accuracy are required, which enable true comparative analysis of kidney function as observed via both invasive and MRI techniques. The combination of invasive techniques and non-invasive MRI in an integrated multi-modality setup with the goal to calibrate, monitor and interpret pMR by means of standardized interventions, is the focus of the present invention.

A system as described herein has not been previously disclosed in the relevant prior art. Beierwaltes et al (Compr Physiol 3:165-200, 2013) disclose a number of potential MRI-based applications regarding the assessment of renal function. An integrated in vivo and MRI-based approach is not disclosed therein.

Schoenberg et al (Radiologe, 2001, 41:146-153) disclose the simultaneous measurement of renal blood flow with an invasive probe in combination with an MRI analysis. The measurement of oxygen saturation of the kidney via an in vivo probe is not disclosed. The setup of Schoenberger represents a technically simplistic device when compared to the present invention. Although blood flow is assessed, no information via oxygen saturation probes is obtained, thereby rendering a comparison between MRI and in vivo measurements of oxygen saturation impossible. A meaningful comparison between in vivo and MRI based analyses of kidney function is not possible with the setup as disclosed on Schoenberger.

A number of disclosures of the prior art are available that describe animal holding devices for MRI analysis (US 2012/0330130 A1, US 2012/0278990 A1, WO 02/32306 A2, WO 2009/015678 A1). None of these documents describe a combined system of invasive and MRI-based analytic possibilities according to the present invention, capable of measuring renal perfusion and renal oxygenation via in vivo physiological measurement and magnetic resonance imaging (MRI). US 2010/0100072 A1 and WO 2012/076660 A1 disclose the possibility of integrating a catheter into the holding device for attachment to the bound subject, in order to provide fluid to the subject. No means for the analysis of kidney function are described.

### SUMMARY OF THE INVENTION

Manifold physics-related phenomena and practical obstacles with respect to the small size scale inherent in invasive analysis represent significant problems in establishing real-time simultaneous in vivo and MRI-based analysis of a mammalian kidney. Practical impediments can be evoked by strong static magnetic fields used for MRI, by time varying magnetic field gradients used for spatial encoding in MRI, by space constraints of small bore MRI scanners, which are tailored for imaging of small rodents and leave a clearance of only few centimetres, as well as by electro-magnetic and acoustic-mechanical interferences between the MRI equipment and the various probes and instruments used for invasive measurements. The development of novel analytical systems requires a careful assessment of MR induced effects and artefacts together with the development of technical solutions that help to overcome compatibility issues between invasive and MRI-based analytical approaches.

In light of the prior art the technical problem underlying the invention was the provision of means to calibrate, monitor and/or interpret parametric MR (preferably MRI) in light of physiological parameters of kidney function (in particular, direct measurements of kidney oxygenation) by means of a standardized and reliable analysis. A further objective of the invention was the provision of means for real-time comparison of MRI-based analytics or diagnostic techniques with corresponding in vivo data from the kidney, thereby allowing conclusions to be drawn regarding the quality of the MRI analysis with respect to potential diagnostic application.

This problem is solved by the features of the independent claim. Preferred embodiments of the present invention are provided by the dependent claims.

Therefore, an object of the invention is to provide a system, method, device or combination of devices, for the simultaneous determination of renal perfusion and renal oxygenation via in vivo physiological measurement and magnetic resonance imaging (MRI) comprising multiple probes for measuring in vivo physiological parameters of kidney function, an animal holding device and an MRI-scanner, preferably a small-bore MRI scanner suitable for carrying out measurements on animals.

The device and system of the invention enable for the first time the in vivo measurement of physiological parameters, in particular direct measurements of kidney oxygenation, and magnetic resonance imaging on a single organ in a single subject.

In a preferred embodiment of the invention the system of the invention comprises one or more near infrared probes suitable for detection or measurement of oxygen saturation and/or haemoglobin concentration. The system may in further embodiments comprise means to support standardized interventions in the MR scanner including hypoxia/hyperoxia or other chemically, biologically or pharmacologically induced interventions. The system may in further embodiments comprise means to support standardized interventions including hypoxia/hyperoxia or other chemically, biologically or pharmacologically induced interventions. Such means may relate to catheters, infusion or intravenous apparatus for providing the subject with any given drug or other treatment. This embodiment enables the testing of kidney medication and real-time assessment of any affects on renal structure or function.

The phrase physiological parameters of kidney function relates essentially to any aspect of renal structure and/or function, for example oxygen saturation or blood flow. For example, kidney size, renal blood flow, local tissue perfusion and blood oxygen content are all major determinants of oxygen delivery to kidney tissue. Each of these factors may be considered physiological parameters and may be assessed by either the in vivo or MRI-based assessment enabled by the present invention. In some embodiments, examples of renal function may relate to any measureable parameter suitable for indication of the state of the kidney and its role in renal function. For example, glomerular filtration rate (GFR) describes the flow rate of filtered fluid through the kidney. In another example, creatinine clearance rate (CCr or CrCl) is the volume of blood plasma that is cleared of creatinine per unit time and is a useful measure for approximating the GFR. The present invention is able to analyse such parameters and others, either directly or indirectly, to provide information on renal function.

In light of the physical modifications carried out to the various probes, animal holders and MRI device as described herein, the invention proposes a novel and inventive contribution to the art. Due to the physical modifications of the devices and measurements systems carried out for the invention and as described herein, the probes can for example also be applied in the analysis of other organs in the body aside from the kidney, whereby oxygen saturation and blood flow are to be assessed.

The invention therefore relates to a system for simultaneous determination of renal perfusion and renal oxygenation via in vivo (physiological) measurement and magnetic resonance imaging (MRI) comprising
- a (preferably perivascular) flow probe for measuring renal blood flow,
- two oxygen probes for measuring cortical and medullary oxygen saturation (partial pressure of oxygen; pO2), respectively,
- an animal holding device, and
- an MRI-scanner, preferably a small-bore animal MRI scanner.

In one embodiment the system of the present invention is characterised in that it comprises no ferromagnetic, paramagnetic or metallic components that are torque inducing (MRI-disrupting). Although various probes described in the art for invasive measurements of renal oxygen perfusion or blood flow may be declared to be MR compatible by their vendors, their compatibility with a 9.4 T scanner has until now been unresolved. The invention relates to novel, specially designed and modified probes, for example the perivascular flow probe for measuring renal blood flow and two oxygen probes for measuring cortical and medullary oxygen saturation (partial pressure of oxygen; pO2), that are MRI compatible at 9.4 T scanner strength. Their compatibility with such high field strengths represents an unexpected and beneficial finding.

In further embodiments of the invention the perivascular flow probe for measuring renal blood flow (and thereby also the system itself) is characterised in that it comprises an acoustic reflector of ceramic material, preferably glass-ceramic. Preferred is macor ceramics, as can be obtained from Transonic Systems, Ithaka, USA.

The various components of the system described herein demonstrate a synergy that provides beneficial results that go beyond the sum of the functions of each of the respective components. For example, the direct comparison of in vivo and MRI-data, in particular with regard to blood flow and/or pO2 measurements, enables a never seen before direct comparison of diagnostically relevant physiological parameters via two distinct methods. Such a comparison provides crucial insights into the diagnostic value of MRI for AKI; a result of significant importance which may be observed as a technical effect that goes beyond the sum of parts. The present invention enables additional testing of the diagnostic capabilities of MRI in the context of any given animal (rodent) model for kidney disease, which is associated with perfusion and/or oxygenation.

The term "determine" in the context of the system and use thereof relates to any kind of assessment or investigation of the given parameters, for example by measurement, detection or identification.

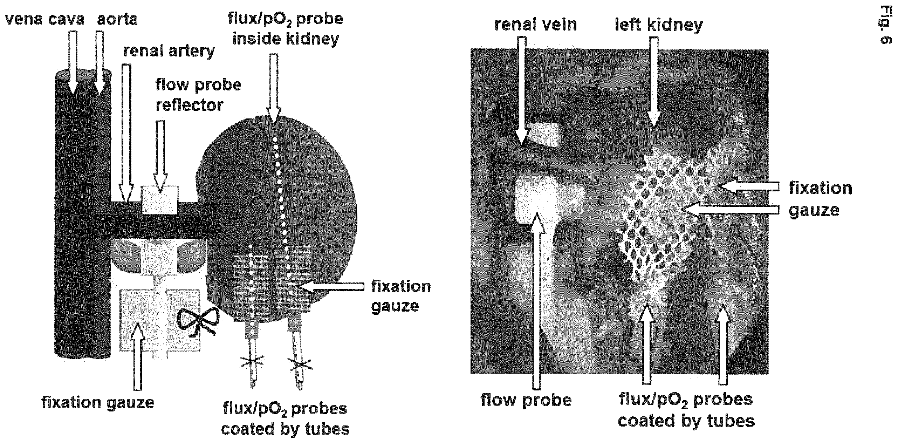

In one embodiment the system as described herein is characterized in that the perivascular flow probe comprises wing-shaped and/or: silicone-reinforced gauze for fixation to tissue. A further embodiment relates to a a perivascular flow probe that is of an L-shaped form. The fragility of the ceramic used in the flow probe requires a thicker and hence larger reflector, which is preferably manufactured in an L-shape design and which preferably lacks a locking device. Unlike the conventional approach, the modified probe of the invention can not be easily attached to the vessel. In one embodiment, since the modified probe can not be positioned from ventral and can not be kept in place by a micromanipulator it must be forwarded from caudal, passing the renal vein dorsally, and positioned with the reflector's opening dorsal onto the artery as illustrated in Figure 6. In order to avoid vertical, horizontal, and axial displacements of the probe wing-shaped silicone-reinforced gauze may preferably be attached to the cable. In one embodiment the system as described herein is characterized in that the perivascular flow probe comprises no vessel locking device. In a preferred embodiment the perivascular flow probe may be a MC2PSB-MRI probe (Transonic Systems Inc., Ithaca, USA) preferably connected to a perivascular flowmeter (T420, Transonic Systems Inc., Ithaca, USA).

In further embodiments of the invention the oxygen probes (and thereby the system comprising said oxygen probes) for measuring oxygen saturation (partial pressure of oxygen; pO2) is characterised in that the oxygen probes are laser-Doppler-flux and/or pO2 probes. The oxygen probes are preferably between 0.1 and 2 mm in diameter, preferably 0.5 or 1 mm.

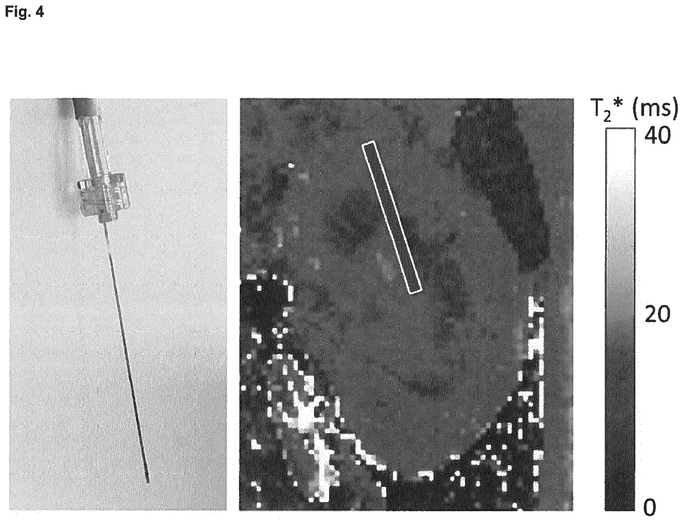

In one embodiment the system as described herein is characterized in that the oxygen probe comprises fibres, wherein said fibers are encased by silicone tubing, which is fitted with a silicone cap, preferably of silicone tape. In a preferred embodiment the distance between the silicone cap of the oxygen probes and probe tip within the silicone tubing is 0.2 to 3 mm, preferably 0.5 to 2 mm, more preferably 1 to 1.5 mm for a cortical probe, or 1 to 6 mm, preferably 2 to 5 mm, more preferably 3 to 4 mm for a medullary probe, respectively. In further preferred embodiments the probes oxygen probes enable local tissue oxygenation and local perfusion measurements by combined optical Laser-Doppler-flux and O2 probes, preferably pO2 E-Series Sensor, Oxford Optronics Ltd, Oxford, UK (Figure 4) preferably attached to an OxyLite/OxyFlo-apparatus (Oxford Optronics Ltd, Oxford, UK).

The system as described herein may additionally comprise means for measuring blood pressure, preferably via a pressure transducer (for example DT-XX, Viggo-Spectramed, Swindon UK) connected to an amplifier apparatus (for example TAM-A Plugsys Transducer, Hugo Sachs Elektronik - Harvard Apparatus GmbH, Mach-Hugstetten, Germany). The blood pressure may be measured via a catheter inserted into the femoral artery. The system is in one embodiment thereby characterised in that one or more catheters are present, for example configured for insertion into the femoral artery and/or femoral vein. In another embodiment the system as described herein comprises an inflatable aortic cuff, preferably of silicone and/or polyethylene material.

The system according to the present invention further comprises an animal holding device which
- is of a half-pipe (semi- or partially cylindrical) shape,
- comprises a radio frequency surface coil array for MRI signal reception,
- comprises a bridge structure for securing the leads for physiological probes, wherein the bridge structure is adjustable in height and distance to the animal, and
- is of suitable dimensions for fitting (after animal and probe mounting) within a small-bore animal MRI scanner, which comprises a magnet bore with a clearance (a space for placing the holder) of a diameter of 15 to 40 cm, preferably 20 cm.

The clearance may in some embodiments be further reduced by the gradient coils used for spatial encoding and shimming and by the radiofrequency coils used for signal transmission. In some embodiments, a linear polarized birdcage resonator was used for transmission that reduces the remaining space to a diameter of less of than 15 cm, for example less than 12 cm, less than 10 cm, in one embodiment of 72 mm.

It was entirely unexpected that the provision of an animal holder for a 300-400 g rat for measurement in a small-bore MRI scanner - and enabling 3 or more in vivo probes - could be provided as disclosed herein. The manufacture of the system as such, in particular due to the size restraints with respect to the animal holder and multiple probes and corresponding cables required, was fraught with significant technical difficulty. The engineering of the required animal holder in its particular dimensions represents a beneficial development that has in part enabled the analysis described herein.

In a preferred embodiment the invention is designed for analysis of a mammalian kidney, preferably of a mouse, rat, rodent or other mammal of similar size, for example those mammals below 400g in mass, preferably below 350g, more preferably around 300 g.

In one embodiment the system as described herein is characterized in that the MRI-scanner comprises a magnet bore with a clearance of a diameter of 15 to 40 cm, 15 to 30 cm, preferably 20 cm. In a preferred embodiment the MRI-scanner has greater than 7 T, preferably a 9.4 T scanner. The set-up described herein is not limited to a small bore 9.4 T MR scanner. It can be transferred to horizontal magnets with even higher magnetic fields; for example 11.7 T. The hybrid approach is also to be expected to be compatible with the new generation of benchtop-size small bore 3.0 T or 1.5 T MR scanners tailored for MRI of small rodents.

In a preferred embodiment, the system is characterized in that the MRI-scanner is configured to carry out and/or performs a T2*/T2 weighted MRI analysis, or that the system comprises a T2* and/or T2 weighted MRI analysis. In further embodiments of the invention the system may be characterized in that the MRI-scanner is configured to carry out or comprises a T2*/T2 weighted MRI, water diffusion MRI, perfusion MR, T1 weighted MRI, renal quantitative susceptibility mapping, kidney size or MR based oxygenation and glomerular function analysis. In yet further embodiments the system as described herein may be characterized in that the MRI-scanner is configured to detail (i. e. interrogate, analyse, measure or determine) the link (for example the relationship or any other kind or proportionality) between renal tissue pO2, renal blood flow, kidney size, conductance, flux and T2*/T2 weighted MRI, water diffusion MRI, perfusion MR, T1 weighted MRI, renal quantitative susceptibility, kidney size or MR based oxygenation and/or glomerular function analysis.

Multiple major challenges appear when integrating invasive techniques with a 9.4 T MR scanner, including: (i) spatial constraints and (ii) keeping ferromagnetic materials outside of the scanner and outside of the close vicinity of the scanner. Considering these requirements, the invention provides new devices and methodology designed to provide appropriate solutions for surgical procedures, surgical instrumentation and experimentation under these conditions.

The instrumentation comprises in a preferred embodiment a femoral artery catheter, a femoral vein catheter, a tracheal cannula, an inflatible suprarenal aortic cuff, a Transonic flow probe positioned around the left renal artery and two combined laser-Doppler-flux-pO2 probes with tips positioned in the cortex and the medulla of the left kidney. Of most importance for the diagnostically relevant analysis are the Transonic flow probe and two combined laser-Doppler-flux-pO2 probes. The additional preferred components enable additional measurements of blood pressure and control of the blood flow to the kidney. To lower renal perfusion pressure, a remotely operated inflatable cuff can be positioned around the aorta right above the renal arteries. For the MRI setting a cuff smaller than that used in conventional settings has been developed. The cuff preferably consists of silicone and polyethylene and is positioned about 15 mm from the kidney in order to not cause artefacts in renal MRI.

The system may also comprise a respiratory mask for the bound mammalian subject. The respiratory mask of the present invention is preferably loosely-fitting to the spontaneously breathing rat's nose. Using this approach, a rat may be supplied with breathing gas mixtures at a preferably flow rate of 1L min-1. The oxygen fraction of the inspired gas mixture (FiO2) is preferably adjusted to 21% (normoxia), 10% (hypoxia), and 100% (hyperoxia). FiO2 can be monitored using a Capnomac AGM-103 (Datex GE, Chalfont St. Gils, UK).

The methodological developments described in the context of the invention represent a time-consuming and technically challenging process which required (in the examples described below) a total number of 20 rats to draw the conclusions referring to the experimental setup and its refinements. The system has however in a surprising fashion facilitated the measurement of various physiological parameters relevant for tissue oxygenation and perfusion inside of a small bore animal scanner. The system described herein supports a number of (patho)physiologically relevant interventions by means of remotely controlled devices within a 9.4 T MRI scanner. Notwithstanding the success of the implementation of the prototype described herein, it should be noted that severe spatial constraints, inter-individual variability of pertinent anatomical features, transfer of the rats from the preparatory lab into the scanner, and the very limited freedom for probe adjustments inside the scanner were and remain challenging aspects of the technology described herein and indicate the unexpected nature of the positive results obtained.

Three hypotheses are the driving force behind the development of the present invention to implement a novel multi-modality approach for the characterization of renal hemodynamics and oxygenation: 1) renal medullary hypoxia and hypoperfusion are crucial factors in the pathogenesis of AKI, 2) combining advanced MRI techniques with modern invasive measurements is feasible, and 3) combined invasive and MRI measurements allow for the calibration and interpretation of pMR results. The examples of the invention provided herein demonstrate the feasibility of an integrated setup, which addresses previous concerns and offsets the commonly held notion of compatibility issues and spatial constraints being prohibitive for such a multi-modality approach.

Adaptation of the experimental equipment was essential to make the entire setup safe for use within the strong magnetic field environment of the MR system. Refinement of the experimental set-up helped to overcome practical obstacles associated with reliable probe positioning, animal transport and sparse space available in a small bore animal scanner. On the MR data acquisition side magnetic gradient switching and application of radio frequency pulses bear the potential to interfere with the invasive measurements. Interferences with the flux signal were observed and should be further investigated to elucidate the root cause and means for artefact elimination. Notwithstanding this interference, unaffected invasive data acquired during silent MR periods would suffice for the hybrid approach.

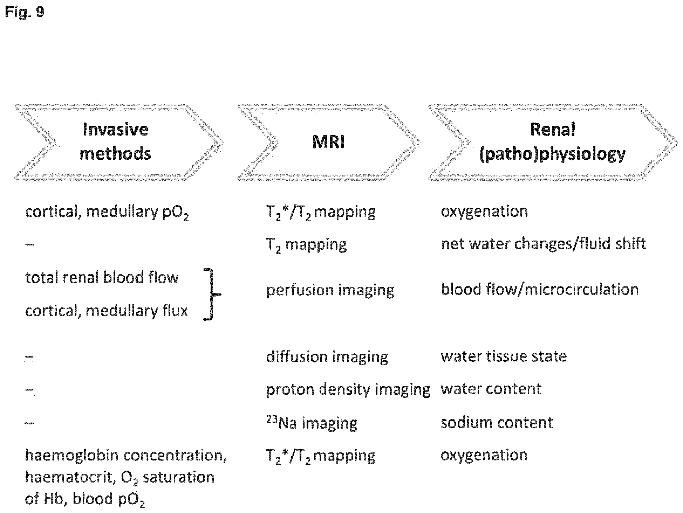

With the proposed setup, as demonstrated in the examples below, renal pMRI was performed simultaneously with invasive measurements of total renal blood flow, cortical and medullary pO2 and flux for the first time *in vivo* in rats. Key technical challenges have been met and standardized interventions have been successfully implemented. This setup may be carried out as part of a renal pMRI analysis system (Figure 9) incorporating preferably T2* mapping to probe for changes in blood oxygenation level and preferably T2 mapping to monitor net water changes and fluid shifts. Additionally, preferably, arterial spin labeling (ASL) for perfusion imaging, proton density imaging to determine total water content, apparent water diffusion coefficient (ADC) determination by diffusion sensitized MRI to probe for water content and water shift, and/or 23 Na imaging to examine renal sodium content are further preferred embodiments of the invention tha may be combined with the setup as described herein.

MR-methodology established for human MRI needed to be carefully adapted to free breathing imaging in rats (with heart rates of approximately 350 beats/min) and hence requires preferably cardiac and respiratory gating to strictly avoid motion effects. This is of particular preference for diffusion weighted imaging and for arterial spin labeling techniques. The latter provides means for MR based assessment of renal perfusion (Wang et al., 2012, Zhang et al., 2012) and can be used for examination of renal blood flow (RBF) values of the cortex and medulla (Liu et al., 2012). Furthermore, the probes for total renal blood flow, flux and pO2 (Figure 9) may be complemented by measurement of blood parameters such as haematocrit, pO2, haemoglobin concentration, and/or O2 saturation of haemoglobin as obtained by intermittent blood samples.

Calibration and validation of pMR will rely on employing invasive methods in *in vivo* studies during various physiological and pathophysiological conditions. Alterations in inspiratory O2 fraction, such as demonstrated here, may help to shed light onto the yet unknown relationship between the surrogate MRI biomarkers and quantitative invasive readouts from one perspective. Other stimuli, such as brief periods of ischemia or administration of adenosine or furosemide could aid to characterize the regulation of renal hemodynamics and oxygenation from other perspectives.

The present set-up can be also employed for longitudinal studies. Chronically instrumented awake animals have been studied previously using appropriate anesthetic regimen for the implantation surgery (Flemming et al., 2000). It should be also noted, that longitudinal studies with the present invention may require adjustments of surgical techniques. These adjustments include fine tuning of probe fixation within the rat, rearrangement of the exit-sites of the probes' leads and their protection against manipulations by the conscious rat.

The present invention demonstrates that integrating invasive physiological methods with MRI is feasible and a comprehensive integrated hybrid set-up is now available. The invention will serve to systematically examine the validity and efficacy of quantitative pMRI as a surrogate marker for tissue properties that are highly relevant to AKI research, such as perfusion and oxygenation. The weakness of MRI, its qualitative nature, can now be addressed by calibration with invasive methods. The strengths of MRI, its non-invasiveness, its suitability for longitudinal studies and its ability to capture the physiological heterogeneity between and within the renal layers, underscore its great potential for research of experimental AKI.

### EXAMPLE 1

### Figures for Example 1:

**Figure 1****:** Basic concept of the proposed hybrid and integrated approach linking quantitative physiological methods with parametric MRI (pMRI).
**Figure 2****:** Flow probes with different acoustic reflectors attached. **Left:** Conventional probe with locking mechanism. **Middle:** Tested probe with larger lumen and equipped with brass reflector. **Right:** Novel probe configuration with ceramic reflector but without locking mechanism.

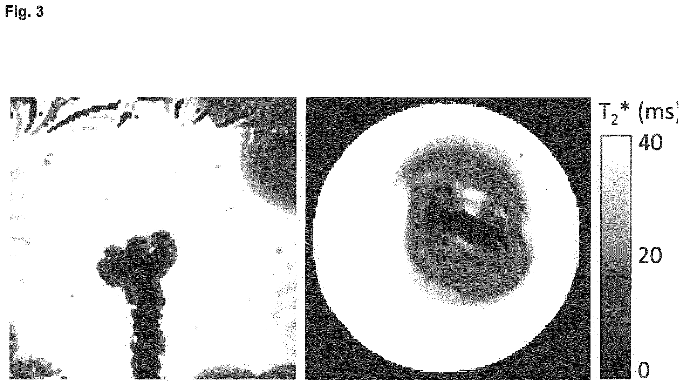

**Figure 3****:** T2* maps of the flow probe **(left)** and of the brass reflector **(right)** embedded in agarose. The high T2* (white) of agarose is reduced in areas with susceptibility artefacts (color or black). Regions lacking water protons do not generate an MR signal and hence appear as signal voids (black) with the area reflecting the real dimensions of the probe and brass reflector. The flow probe is surrounded by a minor susceptibility artefact, which does not interfere with renal MRI since the probe is placed in a safe distance to the kidney. The brass reflector showed severe image artefacts **(right)** extending far beyond its real dimensions.
**Figure 4****:** The 0.5 mm thin combined optical Laser-Doppler-flux and pO2 probes **(left)** was tested for image artefacts *in vivo* by placing it inside the kidney of a rat. In the T2* map obtained for a coronal oblique slice of the kidney **(right)** the probe's signal void manifests itself as a straight line (probe position indicated by white outline). This feature was used to visualize and determine the correct position of the probes. Artefacts beyond the real dimensions of the probe were not observed.
**Figure 5****:** Custom designed animal holder. The animal holder is equipped with a bridge that can be adjusted in height and distance to the animal to fix the leads that connect the physiologicial probe heads with the data acquisition apparatus outside of the scanner room.
**Figure 6****:** Scheme **(left)** and situs **(right)** illustrating the position of the perivascular probe for total renal blood flow and the cortical and medullary flux/pO2 probes together with measures taken to fix probes at their respective positions.
**Figure 7****: Left:** cortical and medullary pO2, flux, and T2*/T2 prior, during and after hypoxia (n=1). The intervention started at t=0 and its duration is indicated by the gray shading. **Top Right:** a T2* map illustrating the difference in T2* between hypoxia and baseline. **Bottom Right:** aT2 map illustrating the difference in T2 between hypoxia and baseline. Areas showing an increase in T2 (red) for the most part collocate with larger blood vessels and are likely to be artefacts due to minor shifts in kidney position during baseline and intervention.
**Figure 8****: Left:** cortical and medullary pO2, flux, and T2*/T2 prior, during and after hyperoxia (n=1). The intervention started at t=0 and its duration is indicated by the gray shading. **Top Right:** aT2* map illustrating the difference in T2* between hyperoxia and baseline. **Bottom Right:** aT2 map illustrating the difference in T2 between hyperoxia and baseline.
**Figure 9****:** Synopsis of a comprehensive and integrated multi-modality approach that combines invasive physiological methods with MRI to advance the capabilities of renal hemodynamics and oxygenation characterization.

### Introduction:

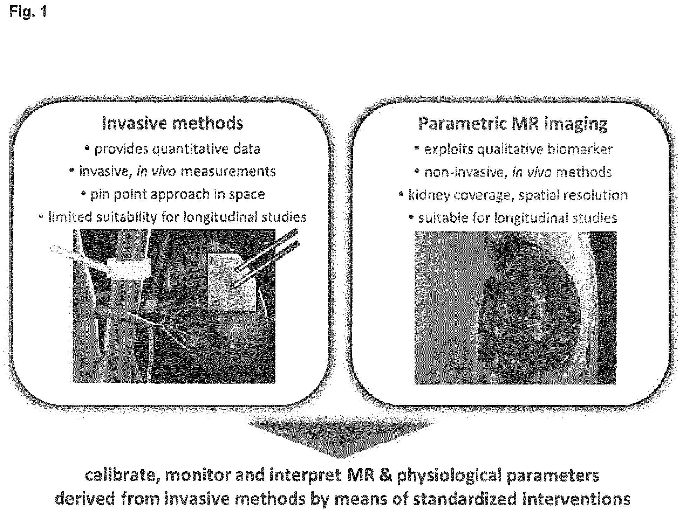

The invention is based on examples that highlight key characteristics of renal perfusion and oxygenation. Concepts for *in vivo* characterization of renal perfusion and oxygenation are surveyed together with the capabilities of Magnetic Resonance Imaging (MRI) for probing blood oxygenation level dependent tissue stages. To this end, two modalities are combined: in vivo physiological methods and non-invasive *in vivo* parametric MRI (pMRI), which is schematically illustrated in Figure 1. Practical concerns evoked by the use of strong magnetic fields in MRI and interferecences between MRI and physiological probes are discussed. Moreover, technical solutions that balance the needs of *in vivo* physiological measurements together with the constraints dictated by space and other challenges inherent to small bore MR scanners are presented. An early implementation of the present approach is demonstrated in an integrated hybrid setup, where brief interventions of hypoxia and hyperoxia are explored. All the animal experiments reported here were carried out in accordance with the guidelines provided and approved by the Animal Welfare Department of the *Landesamt für Gesundheit und Soziales* Berlin (Berlin State Office of Health and Social Affairs) and conform to the "European Convention for the Protection of Vertebrate Animals used for Experimental and other Scientific Purposes' (Council of Europe No 123, Strasbourg, 1993).

### Hybrid Setup: Integration of invasive measurements and MRI

Manifold physics-related phenomena and practical obstacles with respect to the small size scale represent significant problems in establishing real-time simultaneous in vivo and MRI-based analysis of a mammalian kidney. Practical impediments can be evoked by strong static magnetic fields used for MRI, by time varying magnetic field gradients used for spatial encoding in MRI, by space constraints of small bore MRI scanners, which are tailored for imaging of small rodents and leave a clearance of only few centimetres, as well as by electro-magnetic and acoustic-mechanical interferences between the MRI equipment and the various probes and instruments used for invasive measurements.

Small bore animal MR scanners largely exceed the magnetic field strength commonly used in clinical practice. In our hybrid implementation, a magnetic field strength of 9.4 Tesla (Bruker Biospec 94/20, Ettlingen, Germany) was employed. This setup provides a magnetic flux densitiy which is 6 times stronger than that of a clinical 1.5 scanner. This means that an object or device brought into the magnet would experience a magnetic moment (magneto-static torque) which is 6 times larger versus the 1.5 T environment due to the interaction of the MRI static magnetic field with the magnetization in the object/device. The strong static magnetic field dictates special safety measures and compatibility requirements. Equipment and electronics used for invasive measurements (OxyLite apparatus, Oxyflo apparatus, perivascular flowmeter, pressure amplifier, adc converter and a PC to log all physiological signals) that is prone to forces generated by magnetic fields was installed on a trolley and safely placed outside of the scanner room. Alternatively, this equipment can be mounted to the wall or floor of the scanner room to strictly avoid what is been commonly known as a 'missile effect'. In any case, the physiological devices are preferably positioned at least 3 m from the scanners isocenter to avoid magnetic stray field effects, which might hamper the performance of the electronics, amplifiers and devices used for the invasive methods. Consequently, extension lines were added to the physioloigcal probes to bridge the distance between the devices and electronics used for the invasive methods and the isocenter of the magnet.

Although probes used for the invasive measurements may be declared to be MR compatible by the vendors, their compatibility with a 9.4 T scanner is unresolved. To meet this requirement the probes must not contain any ferromagnetic, paramagnetic or metallic parts that might induce torque or severe image artefacts.

For blood pressure measurements, a pressure transducer (DT-XX, Viggo-Spectramed, Swindon UK) connected to an amplifier apparatus (TAM-A Plugsys Transducer, Hugo Sachs Elektronik - Harvard Apparatus GmbH, Mach-Hugstetten, Germany) was used. Blood pressure was measured via a catheter inserted into the femoral artery. Here the use of a long catheter (-1 m) was prudent to place the transducer well outside of the magnet bore, where the magnetic fringe field does not diminish the efficacy of the transducer.

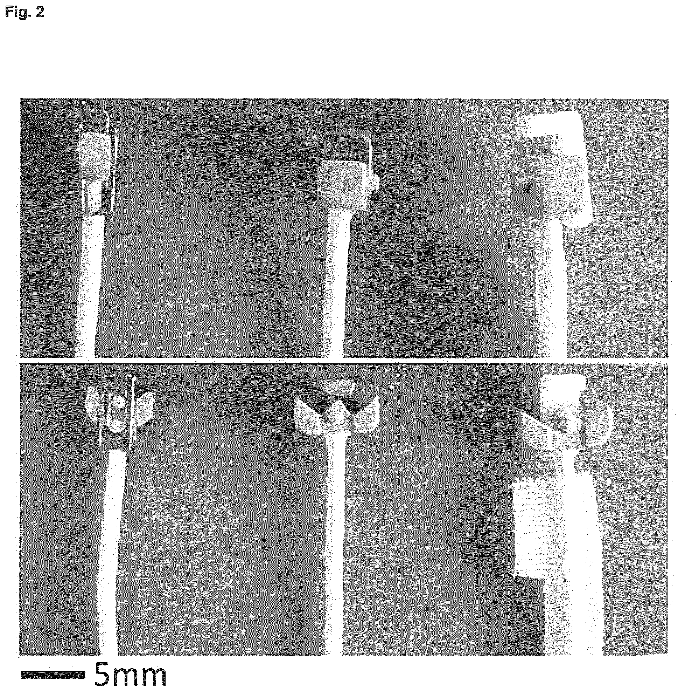

A perivascular flow probe (MC2PSB-MRI, Transonic Systems Inc., Ithaca, USA) connected to a perivascular flowmeter (T420, Transonic Systems Inc., Ithaca, USA) was used to assess total renal blood flow. For this purpose two ultra-sound transducers pass signals back and forth using an acoustic reflector fixed on the probe's body, alternately intersecting the flowing blood in upstream and downstream directions. The resulting time difference is proportional to the blood flow in the vessel. The signal of the conventional probe (Type 1RB, Transonic Systems, Ithaca, NY) was too weak due to the long extension leads. To increase the signal, a larger probe body that houses the ultrasonic transducers was chosen. Also, the conventional probe's steel reflector was replaced by a brass acoustic reflector as illustrated in Figure 2. For assessment of image artefacts, the probe was placed in homogeneous agarose phantoms (2% agarose). MRI was performed using a multi-echo gradient echo technique (MGE) technique (TR/TE/FA = 1330ms / 3.57-34.9 ms (number of echo times=10) / 61°, FOV/ matrix /resolution = 45x45mm / 128x96 / (0.35x0.47) mm2) which is sensitive to susceptibility artefacts and which supports T2* mapping. T2* maps acquired along the long axis of the probe showed an area of signal void that reflects the probe body's real dimensions as demonstrated in Figure 3. The rim around the probe embodies a minor susceptibility artefact that is rather small compared to the probe size. This minor artefact does not interfere with renal MRI of small rodents since the probe is placed around the renal artery in a safe distance to the main target areas in the kidney. The brass reflector, however, revealed severe image artefacts as demonstrated by the distortions and signal void shown in Figure 3. These artefacts are prohibitive for renal MRI in small rodents and hence required replacement of the acoustic brass reflector. To solve this issue a reflector made of macor ceramics (Figure 3) was applied (Transonic Systems, Ithaka, USA). This reflector does not induce artefacts but is L-shaped. It offers no mechanism to lock the vessel which presents a significant challenge for the implantation of the probe.

The flow probe (Transonic) was found to exhibit an offset of 1 ml min-1 in the magnetic field environment. This was confirmed by attaching the transonic probe to a silicone tube which served as a blood vessel model. For calibration and offset assessment the tube was perfused by either blood or saline using a perfusion pump with a range of preset perfusion rates.

For local tissue oxygenation and local perfusion measurements, combined optical Laser-Doppler-flux and pO2 probes (pO2 E-Series Sensor, Oxford Optronics Ltd, Oxford, UK, please see Figure 4) were attached to an OxyLite/OxyFlo-apparatus (Oxford Optronics Ltd, Oxford, UK). Image artefacts induced by the fiber optic probes were expected to be minor due to the small probe diameter of 0.5 mm. The probe was tested *in vivo* by placing it inside the kidney of a rat. Images were acquired (MGE, TR/TE/FA = 100ms / 2.85-28.5ms (10 echo times) / 22°, TA=1:20min) for a coronal oblique slice of the kidney (field of view (FOV) (38.2x50.3) mm2 , matrix 169x215, spatial in-plane resolution (230x230) Vm2 , slice thickness 1.4 mm. The signal void induced by the probe manifests itself as a straight line in the cranial pole of the kidney as demonstrated in Figure 4. This feature was used to visualize and determine the correct position of the probes, a feature not available in the conventional setup of invasive measurements.

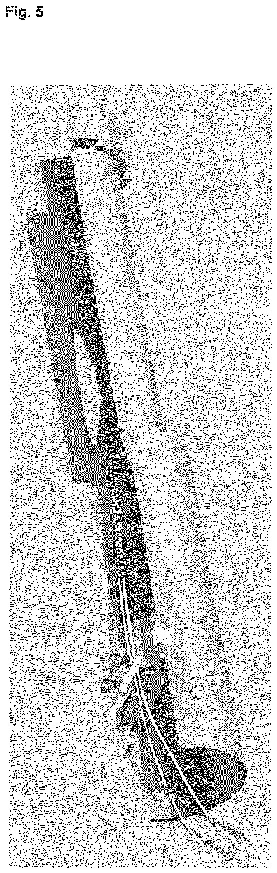

Since surgery is performed outside of the MR scanner room for safety reasons, the transport of the animal into the MR scanner after surgery can be a further obstacle. Every movement of the animal after probe implantation might hold the risk for probe displacement. To fix and stabilize the position of the probes and to accomplish a safe transport of the animal a special animal holder was designed and built (Figure 5) using 3D CAD (Autodesk Inventor 2012, Autodesk Inc., San Rafael, USA) and rapid prototyping (BST 1200es, Alphacam GmbH, Schorndorf, Germany). By carefully considering our initial experience the holder was customized to meet the geometry constraints given by the MR setup. The animal holder has a half pipe shape and accommodates the radio frequency surface coil array used for MRI signal reception. Prior to surgery the animal is placed on the holder in a supine position and does not change its position until the end of the MR examination. A bridge was positioned right behind the hindpaws with the goal to fixate the leads that connect the physiological probe heads with the equipment positioned outside of the MR scanner room. The leads are semi-rigid so that any movement might disarrange the probes. For this reason the bridge can be adjusted in height and distance to the animal. The rigid animal holder in conjunction with the adjustable cable support enable a safe transport of the animal to the MR scanner.

An external standard has been used for control and calibration of the MR techniques employed for T2*/T2 mapping. For this purpose two short capillaries (outer diameter=3 mm, inner diameter=0.4 mm, length=15 mm) were filled with a paramagnetic solution (CuSO4, 0.014 g I-1) and distilled water. For the *in vivo* experiments, both reference standards were positioned in the close vicinity of the left kidney.

After establishing compatibility with a 9.4 T environment, and after transport issues had been solved with the special animal holder, the space constraints of the small bore animal scanner presented a significant practical impediment. The magnet bore has a clearance with a diameter of 20 cm. This valuable commodity is further reduced by the gradient coils used for spatial encoding and shimming and by the radiofrequency coils used for signal transmission. In our implementation, a linear polarized birdcage resonator was used for transmission that reduces the remaining space to a diameter of 72 mm. Further space is consumed by the RF coil used for signal reception which was placed in a laterodorsal position as close as possible to the kidney to achieve highest sensitivity for MRI. Our setup makes use of a four channel receive only rat coil (Bruker Biospin, Ettlingen, Germany) tailored for abdominal imaging that reduces the clearance to a diameter of approximately 60 mm. The animal holder was rotated by about 10-20° around the longitudinal axis to align the kidney with the position of the receive RF coil. A small respiratory pad was placed such that the respiratory motion led to a compression of the pad during inspiration. This pad was connected to a pressure transducer and the resulting physiological signal served to monitor the respiratory motion (P-resp, Model 1025, SA Instruments, New York, USA) and to gate MRI acquisitions. Adding a heating pad further narrowed the space available to the rat, which was then less than 60 mm in diameter. Arguably, all these space constraints have major implications for probe positioning and place special requirements on animal preparation.

### Hybrid Setup: Surgery and Animal Preparation

In our setup, anaesthetized rats are placed in supine position on a temperature-controlled (39°C) operation table and remain there throughout instrumentation and experimentation. Instrumentation surgery and placing of probes is aided by an operation microscope. The exact positioning of various probes is achieved with micromanipulators that also keep the probes in place throughout the experiment. The abdominal cavity remains open throughout the experiment. This approach allows visual control of the kidney and the probes, and enables on-line device adjustments if necessary.

The use of micromanipulators during experimentation is prohibited by space and safety constraints. Therefore, new procedures for the positioning and fixing of invasive probes during surgical preparation were established. To avoid probe displacement during the rat's transfer into the scanner, the rat was placed in the animal holder already during instrumentation. The abdominal cavity was closed after completion of the instrumentation. This approach was essential for placing the heating pad above the ventral abdominal wall. This procedure was dictated by the need for placing the RF receive coil laterodorsal as close as possible to the kidney to preserve MR sensitivity. Because of spatial constraints, preferably rats of a body mass < 350 g are applied as they fit more easily into the available space. As kidney and blood vessel size varies with the BM of rats, the placement of the relatively large Transonic probes was not possible if not elusive for a considerable portion of the smaller rats involved in our preliminary study.

Our instrumentation comprises preferably a femoral artery catheter, a femoral vein catheter, a tracheal cannula, an inflatible suprarenal aortic cuff, a Transonic flow probe positioned around the left renal artery and two combined laser-Doppler-flux-pO2 probes with tips positioned in the cortex and the medulla of the left kidney. Instrumentation and experimentation is done under general anaesthesia (urethane, Sigma Aldrich, Germany, 20% in destilled water, 6 ml kg-1 BM i.p.). Since this approach provides anesthesia for hours and leaves cardiovascular reflexes largely undisturbed, we also used this approach for the present setup. Insertion and fixation of both the femoral artery and vein catheters did not require MR specific adaptation. The positioning of the rat within the isocenter of the MR scanner required an extension of the catheters to about 1200 mm. The dead space volume of the arterial catheter was calibrated in volume and filled with saline. The femoral artery catheter was connected to a pressure transducer positioned outside the magnet bore. A respiratory mask was developed, which was loosely-fitting to the spontaneously breathing rat's nose. Using this approach the rat was supplied with breathing gas mixtures at a flow rate of 1L min-1 . The oxygen fraction of the inspired gas mixture (FiO2) was adjusted to 21% (normoxia), 10% (hypoxia), and 100% (hyperoxia). FiO2 was monitored using a Capnomac AGM-103 (Datex GE, Chalfont St. Gils, UK).

To lower renal perfusion pressure a remotely operated inflatable cuff was positioned around the aorta right above the renal arteries. For the MRI setting a cuff smaller than that used in the conventional setting was prepared. Since the cuff consists of silicone and polyethylen and is positioned about 15 mm from the kidney it does not cause artefacts in renal MRI. To allow visual control of in- und deflation of the implanted aortic cuff it was connected to a control cuff placed outside the scanner bore using a T-branche. In this setting the Transonic flow probe is positioned around the left renal artery. Probe mounting is done by a ventrodorsal movement of the micromanipulator. The steel reflector of this probe is hook-shaped and is locked around the artery by means of a locking device. Thus, the probe is held in place throughout the experiment using the locked reflector and the micromanipulator. For the present setup a probe with a larger body size was used to gain signal. On the downside the anatomical distance between the aorta and the renal hilus (Figure 6) was found not to offer sufficient space for probe placement for approximately 40% of rats involved in the early studies. The use of larger rats (- 350 g BM) helped to eliminate this problem but turned out to be not feasible in the long term run due to the spatial constraints in the scanner. In particular, the bulk of the intestine bears the risk to cause additional pressure, which might enhance the propensity for probe dislocation. This was observed for several rats where the probe exerted pressure on either the aorta, the renal artery and vein, or the kidney itself, which can result in ischemia or congestion of the kidney. In these cases, the flow probe was placed around the renal vein. If this proved not to be feasible the examination was performed without the flow probe being included in the prototype setup.

The flow probe's ceramic reflector gives rise to yet another challenge. The fragility of the ceramic asks for a thicker and hence larger reflector, which can only be manufactured in an L-shape design and which lacks a locking device. Unlike the conventional approach, the modified probe can not be easily attached to the vessel. Since the modified probe can not be positioned from ventral and can not be kept in place by a micromanipulator it was forwarded from caudal, passed the renal vein dorsally, and was positioned with the reflector's opening dorsal onto the artery as illustrated in Figure 6. In order to avoid vertical, horizontal, and axial displacements of the probe a wing-shaped silicone-reinforced gauze was attached to the cable. The gauze was fixed to the retroperitoneal muscles (Figure 6) using single sutures. Since Transonic measurements rely on ultrasound appropriate coupling into tissue is of high relevance. This is achieved by filling the abdominal cavity with saline solution (38 °C). The site where the leads of the Transonic and of the other probes were led out of the abdomen could not be entirely closed by suture. An additional small catheter was placed into the abdominal cavity before the abdomen was closed to replenish saline leakage throughout the course of the experiments.

In our setting the combined laser-flux and pO2 probes are inserted from the ventral surface of the kidney into the renal cortex and medulla. This is done by a micromanipulator, which enables accurate positioning of the probes' tips with regard to its depth from the renal capsule, i.e. within the targeted kidney layer. Usually, the capsule to probe tip distance is adjusted to 1-1.5 mm for the cortical probe while a 3-4 mm distance is used for the medullary probe. The micromanipulator also keeps the probe in place throughout the experiment. Due to respiration-related movements of the diaphragm, the kidney constantly oscillates along its cranio-caudal axis. When inserted from ventral the pliable laser-flux-pO2- probes can follow these movements while keeping the position of the intrarenal probe tips in place.

Integration into the present setup revealed three major challenges. First, the laser-flux-pO2-probes needed to be inserted via the caudal extremity of the kidney due to the spatial constraints as illustrated in Figure 6. With this setup respiration-related kidney movements along its cranio-caudal axis result in constant oscillations of the probes' tips within the kidney if the probes are not fixed to the kidney. Secondly, the exact positioning of the probes' tips within the targeted kidney layer needed to be achieved without the assistance of a micromanipulator. Third, the probes must be fixed so that displacement does not occur during closing of the abdominal wall at the end of the preparation phase. Several approaches were investigated to solve the problem of laser-flux-pO2-probe integration. First, the fibres of the probes were fitted with a silicone tubing of a predetermined length on which a plastic cap was mounted. With this setup the distance between the cap's brim and the probe tips matched the desired depth from the capsule (1-1.5 mm and 3-4 mm, respectively). The brim was fixed on the capsule of the caudal extremity by means of histoacryl glue (Braun Surgical GmbH, Melsungen, Germany). Each probe was equipped with an individual cap. Caps were fixed separately or combined to the renal capsule but the combined weight of the devices resulted in a rotation of the kidney that endangered its perfusion. To solve this issue we inserted one of the probes via a slot in the brim of the other. Using this device, we obtained reliable measurements for the medulla but not for the cortex. The main reason for this failure is that the cap exerted pressure onto the underlying cortical tissue. Also the histoacryl glue may have damaged subcapsular vessels. In the next step we aimed at advancing the tip of the cortical probe tangentially along the lateral or the ventral surface of the kidney in about 1 mm depth from the capsule. Here, the tip eventually lay about halfway from the lower to the upper extremities within the cortex. Again, this approach did not provide reliable data for the cortex. The advancement of the probe bears the risk to damage numerous interlobular arteries and veins which are located perpendicular to the capsule and reach from the cortico-medullary border to the subcapsular tissue. Therefore we decided to advance the cortical probe from the caudal extremity centrally through the kidney all the way to the cortical layer of the cranial extremity as demonstrated in Figure 6. Since the direction of probe advancement is parallel to the interlobular vessels the risk to damage these vessels is markedly reduced versus our early attempts. In order to position the probe's tip accurately within the cranial cortex this approach requires that the diameter between the caudal and the cranial extremities in each individual kidney is measured by a caliper gauge. Based on this measurement the probes are carefully prepared so that the distance between the insertion point and the tip exactly matches the individual kidney's diameter minus 1.5 mm. In addition, the probe must be advanced meticulously along the caudo-cranial axis in order to prevent it from piercing through the capsule. Although this approach can not be expected to succeed in all rats *per se* it proved to be the most successful approach with regard to reliable cortical measurements.

MRI experiments in phantoms revealed that the plastic cap used for probe fixation caused MRI artefacts. The caps were replaced by small strips of slim silicone tape with tailored patches of gauze fixed to one end and attached to the other end of the silicone tubing that covers the fiber outside the kidney. The gauze was fixed on the capsule of the kidney's ventral surface by means of histoacryl glue (Figure 6). With these solutions in place we sought to fix the probes to avoid any probe displacement during the re-closing of the abdominal wall. Owing to the spatial constraints the customary Luer-Lock connectors were removed and the probes' fibre glass cores were fixed within its customary tubing by means of brass screws. The two probes' tubing was fixed by sutures to retroperitoneal muscles as displayed in Figure 6. The probes extensions were led through the abdominal wall using a small incision in the left inguinal region. The line for the aortic cuff, for the abdominal saline flushing, the cables for the Transonic probe and the temperature probe were led through the caudal cutting edge of the median abdominal incision, and the abdominal wall was closed by a continuous suture.

### Proof-of-Concept In Vivo Study

The feasibility of the present setup was examined in 3-4 months old Wistar rats with a body mass of 300-350g. For this purpose eight rats were studied; five of which could be successfully equipped with a transonic probe for RBF measurements. The average time for surgical preparation was 2 hours. T2* mapping was performed using a multi-gradient multi echo (MGE) technique (repetition time = 50 ms, number of echo times = 10, first echo time = 1.43 ms, echo increment = 2.14 ms, averages = 4) with a total acquisition time of approx. 1 min 20 s (respiratory rate dependent). T2 mapping was conducted using a multi spin echo (MSME) sequence (repetition time = 550 ms, number of echo times = 7, first echo time = 10 ms, echo increment = 10 ms, averages = 1) resulting in total acquisition time of approx. 1 min 40 s. A coronal oblique image slice was acquired (spatial in-plane resolution of (226 x 445) ^m2 , FOV= (38.2 x 50.3) mm3 , matrix size = 169 x 113 zero-filled to 169 x 215 elements, slice thickness = 1.4 mm for MGE and 1.5 mm for MSME). Local B0 shimming that uses a voxel enclosing the kidney only was performed prior to T2*/T2 mapping to improve B0 uniformity. In doing so, the susceptibility weighting is dictated by microscopic B0 susceptibility gradients, rather than by macroscopic B0 field inhomogeneities. Shim currents were kept constant throughout the course of the experiment. An external standard was used as a T2* reference.

Short periods of hypoxia and hyperoxia were used as test stimuli. They were induced by changing the inhaled gas mixture to 10% oxygen concentration (hypoxia) and 100% oxygen concentration (hyperoxia) for approximately 12 min. Prior to intervention T2* and T2 maps were acquired interleaved for a baseline of approx. 15 min. Post intervention T2* and T2 mapping was carried out for approx. 15 min. During intervention T2* maps were acquired 1 minute and 6 minutes after begin of the intervention and T2 maps were acquired at 3 minutes and 8 minutes. The invasive parameters arterial blood pressure, renal blood flow, cortical and medullary pO2 and flux were logged continuously with a sampling rate of 1Hz (Data Acquisition Hardware, Hugo Sachs Elektronik - Harvard Apparatus GmbH, Mach-Hugstetten, Germany).

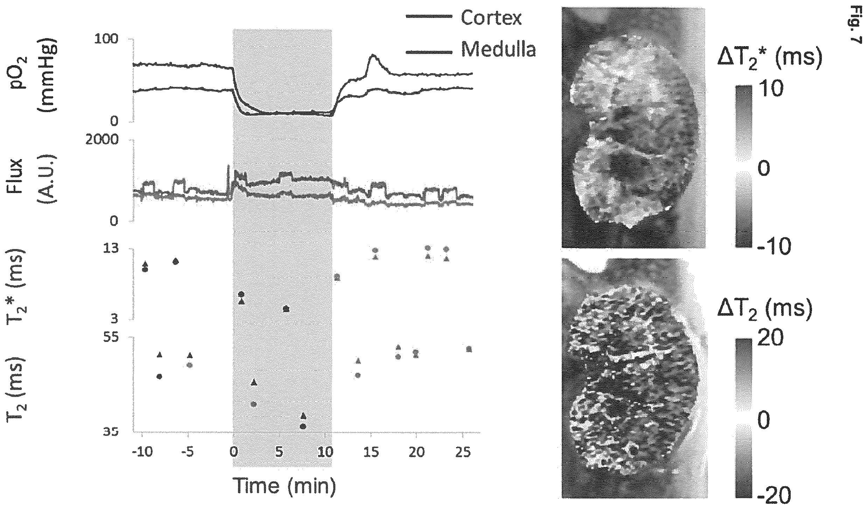

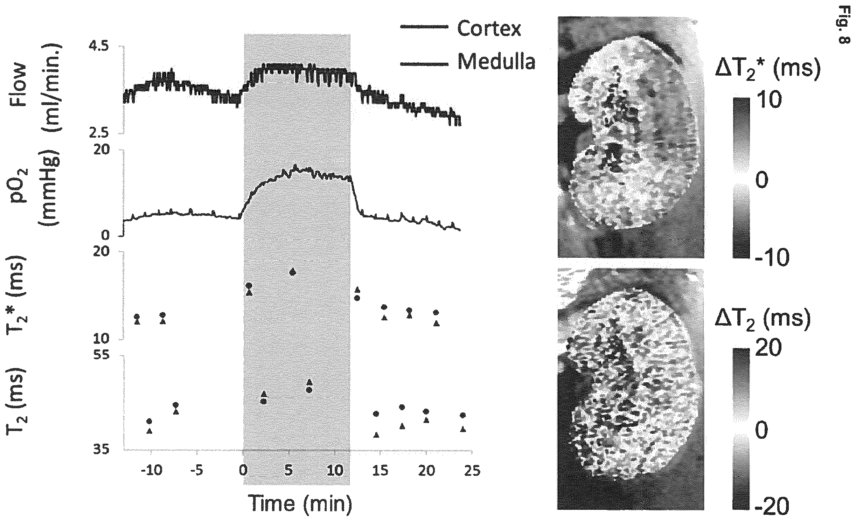

Figure 7 and Figure 8 provide preliminary results derived from simultaneous invasive measurements and MRI in two rats. These data show the response to hypoxia in one rat, and the response to hyperoxia in another animal. At onset of hypoxia, medullary and cortical pO2 and T2* decreased substantially. Medullary and cortical flux increased in response to hypoxia. During hyperoxia cortical pO2, medullary and cortical T2* and total renal blood flow increased simultaneously. All parameters returned to baseline within 15 minutes of recovery. T2* and T2 difference maps shown in Figure 7 and Figure 8 demonstrate that the decrease/increase of T2* and T2 for hypoxia/hyperoxia is rather uniform across the entire kidney. Closer inspection of the flux signal trace in the hypoxia experiment revealed signal elevations that correlated with the start and duration of the MR scans. This can be attributed to an interference with MRI and might be caused by vibrations related to the fast switching of MR gradients. The pO2 probes show small peaks that correlate with the beginning of each MR scan. Measurements performed during MR silent periods were free of signal trace distortions for all probes. These preliminary results demonstrate that simultaneous measurement of pO2, flux, renal blood flow, arterial blood pressure and MRI is feasible in the proposed setup.

### EXAMPLE 2

Objectives: This study is designed to detail the relation between renal T2* and renal tissue pO2 using an integrated approach that combines parametric MRI and quantitative physiological measurements as described herein.

Materials and Methods: Experiments were performed in 21 male Wistar rats. In vivo modulation of renal hemodynamics and oxygenation was achieved by brief periods of aortic occlusion, hypoxia and hyperoxia. Renal perfusion pressure (RPP), renal blood flow (RBF), local cortical and medullary tissue pO2 and blood flux were simultaneously recorded together with T2*, T2 mapping and MR based kidney size measurements (MR-PHYSIOL). MRI was carried out on a 9.4 Tesla small animal MR system. Relative changes in the invasive quantitative parameters were correlated with relative changes in the parameters derived from MRI using Spearman's analysis and Pearson's analysis.

Results: Changes in T2* qualitatively reflected tissue pO2 changes induced by the interventions. T2* versus pO2 Spearman rank correlations were significant for all interventions, yet quantitative translation of T2*/pO2 correlations obtained for one intervention to another intervention proved not appropriate. The closest T2*/pO2 correlation was found for hypoxia & recovery. The inter-layer comparison revealed closest T2*/pO2 correlations for the outer medulla and showed that extrapolation of results obtained for one renal layer to other renal layers must be made with due caution. For T2* to RBF relation significant Spearman correlations were deduced for all renal layers and for all interventions. T2*/RBF correlations for cortex and outer medulla were even superior to those between T2* and tissue pO2. The closest T2*/RBF correlation occurred during hypoxia & recovery. Close correlations were observed between T2* and kidney size during hypoxia & recovery and for occlusion & recovery. In both cases, kidney size correlated well with renal vascular conductance, as did renal vascular conductance with T2*. Our findings indicate that changes in T2* qualitatively mirror changes in renal tissue pO2 but are also associated with confounding factors including vascular volume fraction and tubular volume fraction.

Conclusions: Our results demonstrate that the present system is instrumental to detail the link between renal tissue pO2 and T2* in vivo. Unravelling the link between regional renal T2* and tissue pO2 - including the role of the T2* confounding parameters vascular and tubular volume fraction and oxyHb dissociation curve - requires further research. These explorations are essential before the quantitative capabilities of parametric MRI can be translated from experimental research to improved clinical understanding of hemodynamics/oxygenation in kidney disorders.

### Figures for Example 2:

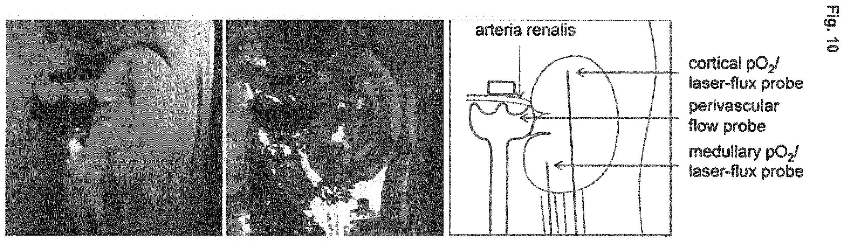

**Figure 10****:** Coronal T2* weighted image of a rat kidney (left) used for control of the perivascular flow probe and the cortical and medullary Laser-flux/pO2 probes position during the in vivo experiments. For this purpose the imaging slice was positioned such that both probes were located within the imaging plane. The T2*map obtained for the same coronal view of the kidney map (middle) also shows the position of the perivascular flow probe and the cortical and medullary Laser-flux/pO2 probes. Schematic view of the positions used for the perivascular flow probe and the cortical and medullary Laser-flux/pO2 probes (right).

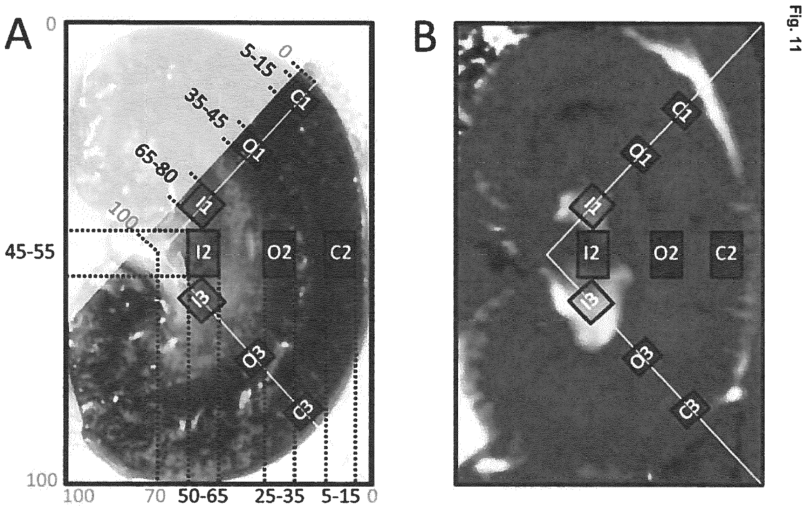

**Figure 11****:** Kidney segmentation model overlaid onto a photograph of a freshly excised rat kidney in coronal view (A) and superimposed to a T2*-map of a rat kidney (B). During analysis the rectangular reference frame is manually positioned around the kidney, followed by an automated drawing of the diagonals (yellow). After their intersections with the kidney borders are defined manually, the ROIs (I1-I2, O1-O3, C1-C3, I: inner medulla, O: outer medulla, C: cortex) are automatically placed at pre-defined relative positions with regard to these references. The numbers shown on the horizontal and vertical axis as well as on the diagonals signify percentages of the reference frame dimensions and of the diagonals.

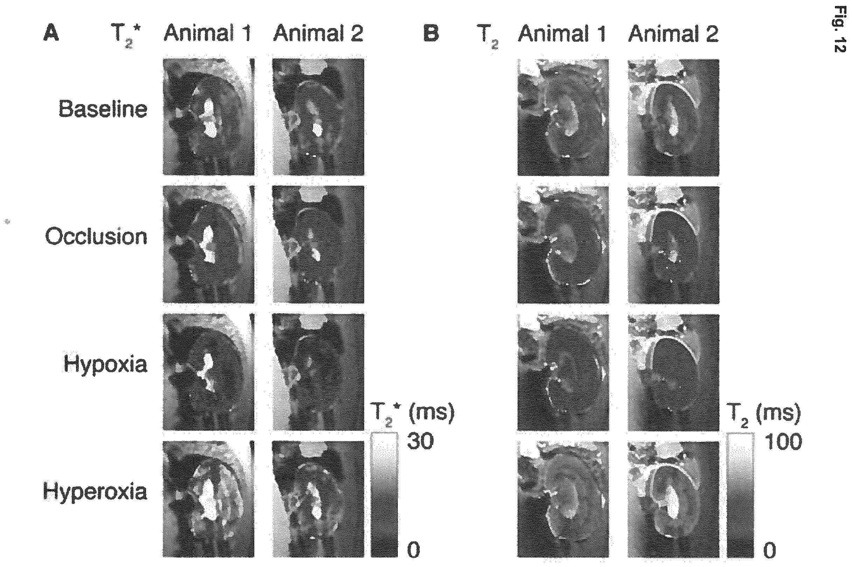

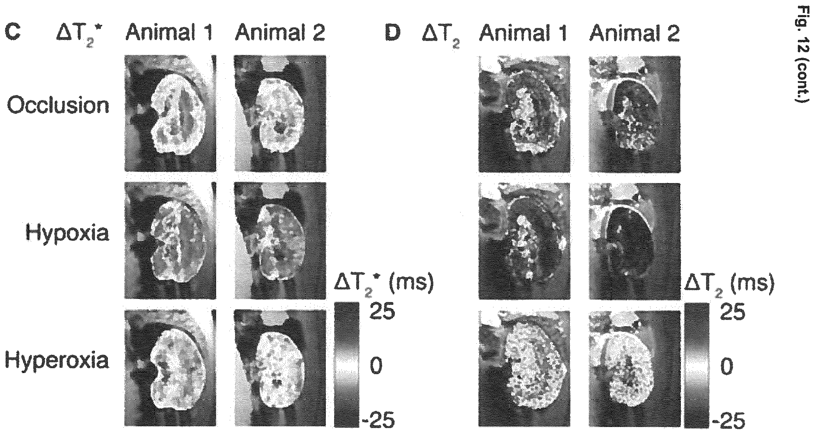

**Figure 12****:** A,B) Examples of renal T2* maps (A) and T2 maps (B) derived from baseline and during aortic occlusion, hypoxia and hyperoxia. C,D) Corresponding ΔT2* (C) and ΔT2 (D) maps which represent the pixel-by-pixel T2* and T2 difference between the last time point of each intervention phase and baseline. Time courses of physiological and MR parameters throughout baseline, hyperoxia and recovery: relative changes of RPP (n=15), RBF (n=10), renal conductance (n=10), kidney size (n=15), cortical/medullary Laser-flux (n=11/13), cortical/medullary pO2 (n=12/10), cortical/outer medullary/inner medullary T2* (n=15/15/15), and cortical/outer medullary/inner medullary T2 (n=14/15/14). Hyperoxia started at t = 0. Its duration is indicated by the grey shading. Absolute parameter values at baseline are used to provide quantitative guidance.

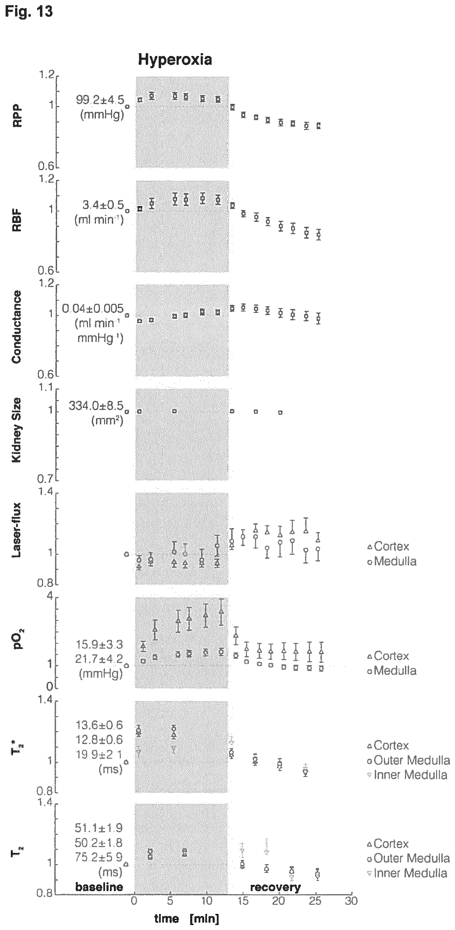

**Figure 13****:** Time courses of physiological and MR parameters throughout baseline, aortic occlusion and recovery: relative changes of RPP (n=15), RBF (n=10), renal conductance (n=10), kidney size (n=15), cortical/medullary Laser-flux (n=12/13), cortical/medullary pO2 (n=12/9), cortical/outer medullary/inner medullary T2* (n=15/15/15), and cortical/outer medullary/inner medullary T2 (n=14/15/14). Aortic occlusion started at t = 0. Its duration is indicated by the grey shading. Absolute parameter values at baseline are used to provide quantitative guidance.

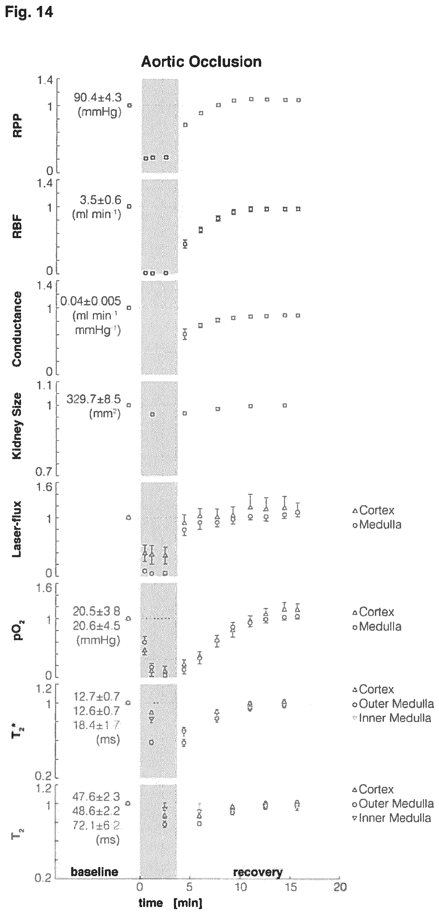

**Figure 14****:** Time courses of physiological and MR parameters throughout baseline, hypoxia and recovery: relative changes of RPP (n=13), RBF (n=8), renal conductance (n=8), kidney size (n=13), cortical/medullary Laser-flux (n=11/13), cortical/medullary pO2 (n=9/8), cortical/outer medullary/inner medullary T2* (n=13/13/13), and cortical/outer medullary/inner medullary T2 (n=12/13/12). Hypoxia started at t = 0. Its duration is indicated by the grey shading. In two rats the hypotensive response was so pronounced that hypoxia had to be stopped prematurely, therefore their data are not included here. Absolute parameter values at baseline are used to provide quantitative guidance.

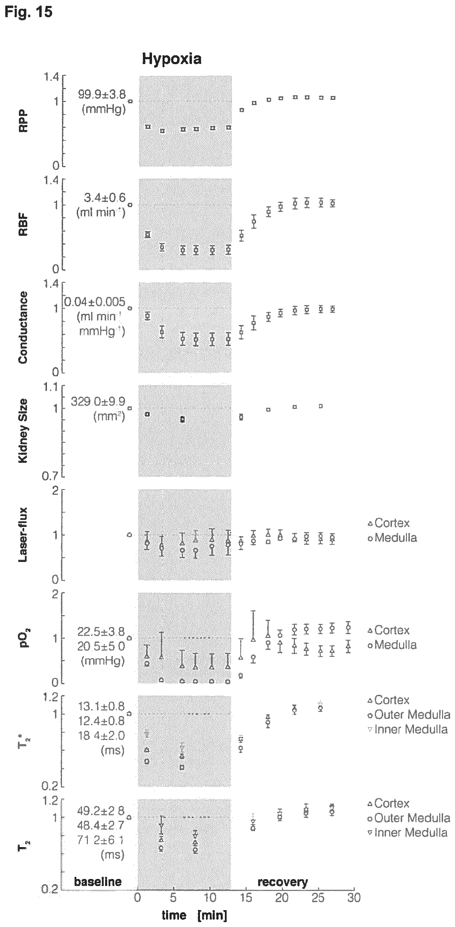

**Figure 15****:** Parametric correlation analysis (Pearson's analysis) between relative changes of cortical T2* and relative changes of cortical pO2 (left) or renal blood flow (right) for aortic occlusion & recovery (top), hypoxia & recovery (center) and hyperoxia & recovery (bottom). The coefficient of determination for Pearson (Rp 2) is given together with the Spearman coefficient (Rs 2); ** denotes p<0.01 The linear regression curve is shown only for significant Pearson's correlations of p<0.05.

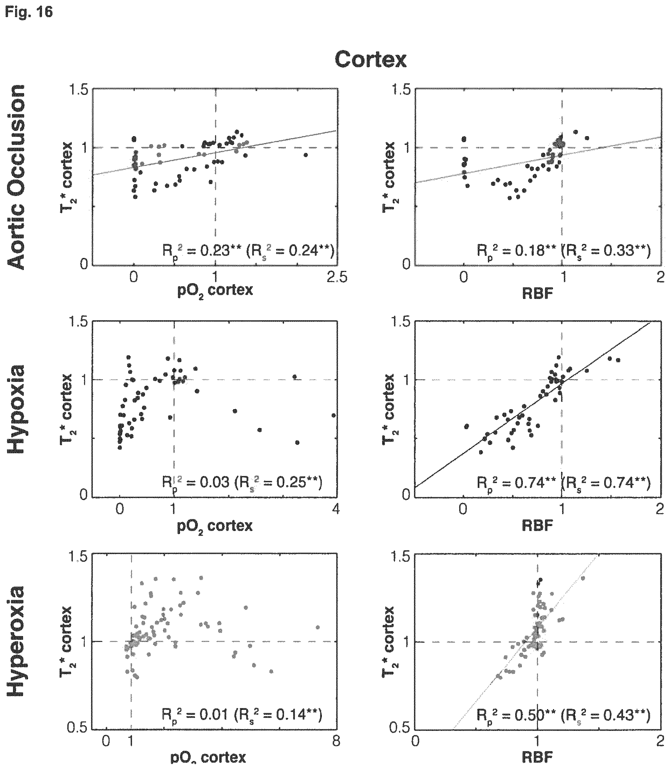

**Figure 16****:** Parametric correlation analysis (Pearson's analysis) between relative changes of outer medullary T2* and relative changes of medullary pO2 (left) or renal blood flow (right) for aortic occlusion & recovery (top), hypoxia & recovery (center) and hyperoxia & recovery (bottom). The coefficient of determination for Pearson (Rp 2) is given together with the Spearman coefficient (Rs 2); ** denotes p<0.01.

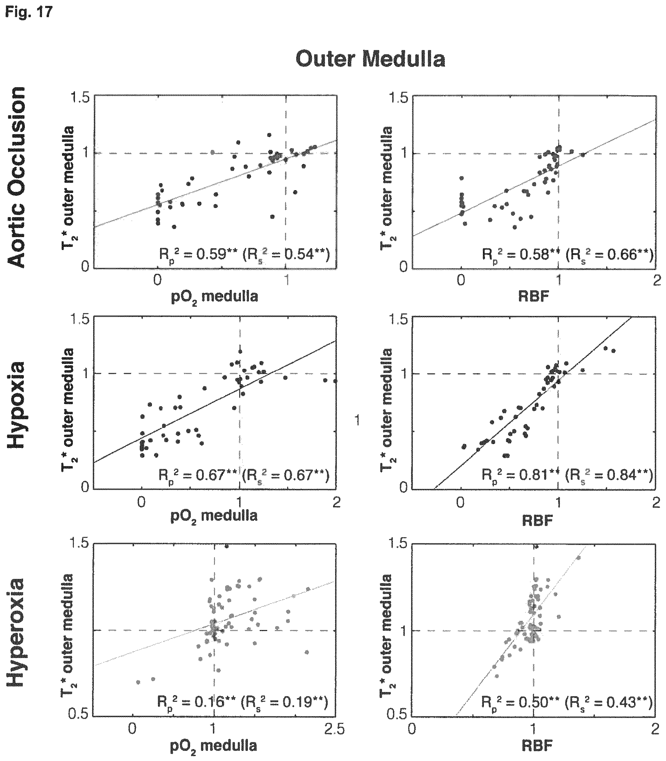

**Figure 17****:** Parametric correlation analysis (Pearson's analysis) between relative changes of inner medullary T2* and relative changes of medullary pO2 (left) or renal blood flow (right) for aortic occlusion & recovery (top), hypoxia & recovery (center) and hyperoxia & recovery (bottom). The coefficient of determination for Pearson (Rp 2) is given together with the Spearman coefficient (Rs 2); * denotes p<0.05, ** denotes p<0.01. The linear regression curve is shown only for significant Pearson's correlations of p<0.05.

### Introduction to Example 2:

Renal tissue hypoperfusion and hypoxia are considered to be pivotal links in the pathophysiological chain of events that leads to acute kidney injury (AKI) as well as the one that promotes progression from AKI to chronic kidney diseases (CKD) [1-7]. Imbalance between renal oxygen supply and demand also appears to play a prominent role in the pathophysiology of diabetic nephropathy [8, 9]. Making ultimate statements on the role of renal hypoperfusion and hypoxia for these renal disorders is elusive since in vivo assessment of renal hemodynamics and oxygenation constitutes a challenge. All modalities available in today's experimental and translational research practice have inherent shortcomings and methodological constraints [2, 10-12].

Obtaining insights into renal perfusion and oxygenation under (patho)physiological conditions by means of non-invasive diagnostic imaging is conceptually appealing. Blood oxygen level dependent (BOLD) magnetic resonance imaging (MRI) and quantitative parametric mapping of the MR relaxation times T2* and T2 are thought to provide surrogates of renal tissue oxygenation in preclinical and clinical studies [10, 12-35]. This assumption is based upon the T2*/T2 dependence on O2-saturation of hemoglobin (Hb) and motivated by the link between O2-saturation of Hb, blood partial pressure of O2 (pO2) and tissue pO2. Although T2* and T2 basically reflect the amount of deoxyHb while pO2 represents the concentration of oxygen, changes in renal T2*/T2 and tissue pO2 may be closely related. Yet their link is also known to be sensitive to the oxyHb dissociation curve, haematocrit, and to the vascular volume fraction [2, 11, 36-38]. This added complexity confounds interpretation of BOLD weighted MRI and parametric T2* mapping data obtained from patients with AKI and CKD [11, 39-41]. For an unambiguous physiological interpretation of renal T2* a calibration with quantitative physiological measurements including renal tissue pO2 is required. For this purpose an integrative multi-modality approach is essential - as underlined in a recent call for further explorations into hemodynamic influences on kidney oxygenation [4] - before the capabilities of parametric MRI can be translated from experimental research to an improved clinical understanding of hemodynamics/oxygenation in AKI and CKD.

An integrated approach that combines parametric MRI with quantitative physiological measurements is prudent to detail the link between renal T2* and renal tissue oxygenation. Quantitative characterization of renal hemodynamics and oxygenation comprises very well established invasive methods that assess perfusion of the entire kidney, regional perfusion and regional oxygenation in the intact animal [2, 12, 42]. MRI offers full kidney coverage, (sub)millimeter spatial resolution, (sub)minute temporal resolution and support of longitudinal studies [10, 12-18, 39, 43]. The validity and efficacy of parametric MRI for quantitative and spatiotemporal characterization of renal tissue perfusion and oxygenation under different functional conditions has not been systematically examined yet and remains to be established. A very limited number of studies attempted to draw a link between established invasive methods and T2*-weighted MRI [44-46]. These studies relied on either comparing MRI and invasive physiological measurements performed in independent cohorts of animals [45, 46] or on T2* and pO2 recorded in the same animal but at different times [33] or in different kidneys (contralateral vs. ipsilatereal) [47].

Realizing the challenges and opportunities of using an integrated multi-modality approach for detailing the relation between renal T2*/T2 and renal oxygenation we hypothesized that simultaneous tracking of invasive physiological parameters and MR parameters derived from the same kidney will elucidate to which extent alterations of physiological parameters are reflected by changes in renal T2* and T2. To test this hypothesis an integrative hybrid approach was employed that combines established invasive measurements including renal perfusion pressure, renal blood flow, local blood flux and tissue pO2 with T2*, T2 mapping and MR based kidney size measurements (MR-PHYSIOL) [12]. In vivo modulation of renal hemodynamics and oxygenation was achieved by standardized (patho)physiologically relevant but reversible interventions, including brief periods of aortic occlusion, hypoxia and hyperoxia. Relative changes in the invasive quantitative parameters were correlated with relative changes in the parameters derived from MRI.

### Materials and Methods:

### Animal Preparation:

All investigations were approved by the Animal Welfare Department of State Office of Health and Social Affairs in accordance with the Animal Protection Law. The spatial constraints dictated by the MR environment required the use of relatively small rats. For this reason, experiments were performed in 21 male Wistar rats (aged 27 g; Harlan-Winkelmann, □12-13 weeks, body mass (BM) 336 Borchen, Germany). The animals were allowed ad libitum food (standard diet) and water and were housed under standard conditions with environmental enrichment. For anesthesia urethane (20% in distilled water; 6 mL kg-1 BM i.p.; Sigma-Aldrich, Steinheim, Germany) was used throughout the surgical preparation and the MRI examination. This approach provides anesthesia for several hours and leaves cardiovascular reflexes largely undisturbed. Body temperature was maintained at 37 °C by means of a mat through which warm water circulates.

Monitoring of absolute arterial blood pressure that corresponds with renal perfusion pressure (RPP; in mm Hg) was achieved by placing a catheter into the femoral artery with its tip towards the aorta [12]. The catheter was connected to a pressure transducer (DT-XX, Viggo-Spectramed, Swindon, UK) and amplifier (TAM-A Plugsys Transducer; Hugo Sachs Elektronik - Harvard Apparatus GmbH, Mach-Hugstetten, Germany).

Absolute measurement of renal blood flow (RBF; in mL min-1) used an ultra sound transit time flow probe (MC2PSB-MRI, Transonic Systems Inc., Ithaca, USA), equipped with a customized ceramic reflector, positioned around the left renal artery. Extra care was taken for the probe positioning [12] since pressure of the relatively large flow probe on the aorta, the renal artery and vein and/or the kidney itself bears the risk to cause ischemia or congestion of the kidney. In order to prevent these complications RBF values and the overall condition of the kidneys (e.g., surface coloring and its homogeneity) were carefully monitored during the entire preparation. After positioning of the animal in the MR scanner a low renal T2* (in particular medullary T2*) was used as an additional criterion for early detection of any flow probe-induced renal ischemia. In two cases the flow probe was alternatively placed around the renal vein. In three animals even this probe position was not feasible so that the probe was omitted entirely. To achieve appropriate coupling of the ultrasound flow probe to the tissue, the abdominal cavity was filled with saline via a catheter (38°C; replenished throughout experiment).

Renal vascular conductance [ml min-1 mmHg-1] (the inverse of resistance) was calculated by dividing RBF [ml min-1] by RPP [mmHg]."

Measurement of absolute tissue pO2 (in mm Hg) and erythrocyte flux (arbitrary units) was enabled by combined laser-Doppler-flux/pO2 probes (pO2 E-Series Sensor; Oxford Optronics, Oxford, UK) that were inserted into the renal tissue. One probe was placed in the medullary region, at 4-mm depth. Another probe was placed in the cortical region by advancing it from the caudal extremity, centrally through the kidney to the cortical layer of the cranial extremity [12].

For induction of aortic occlusion during the MR study a remotely operated inflatable cuff was positioned around the aorta right above the renal arteries [12]. Core body temperature was monitored by means of a fiber-optic temperature probe (T1 S-02-B05, Neoptix, Quebec, Canada) placed in the rectum.

The flux/pO2 probe extensions were passed through the abdominal wall using a small incision in the left inguinal region. All other cables together with the lines for the aortic cuff, for saline supply, cables of the perivascular flow probe and fiber-optic connection of the body temperature probe were passed through the caudal cutting edge of the median abdominal incision. The abdominal wall was closed by a continuous suture. For a more detailed description and discussion of the probe implantation and fixation please refer to [12].

### MR Imaging and Parametric Mapping:

MR imaging was carried out on a 9.4 Tesla small animal MR system (Bruker Biosec 94/20; Bruker Biospin, Ettlingen, Germany) equipped with a linear polarized birdcage volume resonator used for RF transmission in conjunction with a curved four channel receive surface RF coil array (Bruker Biospin, Ettlingen, Germany) customized for rats. T2 weighted pilot scans for geometrical planning and slice positioning were acquired first. Local volume selective shimming of the magnetic field homogeneity on a voxel accommodating the kidney only was conducted using an automatic optimization algorithm based on FID length. To visualize the position of the pO2 and Laser-flux probes 3D multi gradient echo (MGE) imaging of the entire kidney was performed (repetition time = 20 ms, echo time = 2.85 ms, total acquisition time = 2 min 55 s, field of view (FOV) = (38.2 x 48.5 x 21.9) mm3, matrix size = 126 x 120 x 72, spatial resolution = (303 x 404 x 304) µm3). The same 3D image set was used for slice positioning applied in the parametric mapping protocols.

Interleaved T2* and T2 mapping were performed with respiratory gated (Model 1025, SA Instruments, New York, NY, USA) imaging protocols. For T2* mapping a multi gradient echo (MGE) sequence (repetition time = 50 ms, number of echoes = 10, first echo time = 1.43 ms, echo time increment = 2.14 ms, averages = 4) with a total acquisition time of 1 min 20 s was used. T2 mapping employed a multi-echo spin-echo (MSME) sequence (repetition time = 550 ms, number of echoes = 7, first echo time = 10 ms, echo spacing 10 ms, averages = 1), resulting in a total acquisition time of 1 min 40s. A coronal oblique slice was placed across the kidney so that the cortical and medullary pO2 and Laser-flux probes were located within the imaging plane as illustrated in Figure 10. An in-plane spatial resolution of (226 x 445) µm2 (FOV = (38.2 x 50.3) mm2, matrix size = 169 x 113 zero-filled to 169 x 215) and a slice thickness of 1.4-1.5 mm were employed.

The presence/absence of blood flow in the major renal blood vessels was confirmed with time-of-flight (TOF) MR angiography (MRA) performed at baseline and immediately after onset of aortic occlusion and onset of reperfusion. For TOF-MRA a spoiled gradient echo technique (2D FLASH, TR = 11 ms, TE = 3 ms, flip angle = 80 degree) with a spatial in-plane resolution of (200 x 268) µm2) and 15 slices (slice thickness = 1.0 mm) was applied.

### Experimental Protocol and Standardized Reversible Interventions:

Throughout the experiments the rats were continuously provided with air (or other gas mixtures) at a rate of 1000 mL min-1 provided by a respiratory mask placed around the muzzle of the spontaneously breathing rat. Following positioning of the rat in the isocenter of the MR scanner, scanner adjustments and slice positioning was conducted followed by baseline T2* and T2 mapping. Subsequently, short-term reversible interventions were performed: hyperoxia, aortic occlusion, and hypoxia, each followed by recovery.

Aortic occlusion was initiated by inflating the remotely controlled suprarenal aortic occluder and verified by absence of the flow-based blood signal in renal TOF-MRA. Occlusion lasted 3 minutes. One set of T2*/T2 maps was acquired before the occluder was deflated to reestablish renal blood flow. The recovery period comprised 9 minutes and three sets of T2*/T2 mapping.

Hypoxia was induced by decreasing the inspiration fraction of oxygen (FiO2) to 8% via changing the gas flow through the respiratory mask to 8% O2/92% N2. FiO2 was monitored using a Capnomac AGM-103 (Datex GE, Chalfont St. Gils, UK). T2*/T2 mapping were performed twice during hypoxia. The first set was acquired immediately after onset of hypoxia, the second set was started 5 minutes after onset of hypoxia. Following hypoxia of approximately 12 minutes FiO2 was restored to 21% (normoxia, room air). The recovery period of 15 minutes comprised four sets of T2*/T2 maps. Hyperoxia was induced using the same protocol, with the exception that FiO2 was increased to 100% by changing the gas mixture to pure oxygen.

Throughout the experiment invasive physiological parameters RPP, RBF, cortical and medullary tissue pO2 and Laser-flux were simultaneously monitored together with T2*, T2 and kidney size derived from MRI.

### Data Processing and Analysis

Parametric maps of T2* and T2 were calculated by pixel-wise mono-exponential fitting to the signal intensities derived from a series of T2* and T2 weighted images acquired at different echo times (in-house developed program; MATLAB, R2010a, MathWorks, Natick, WA, USA).

Kidney movement throughout the experiment was corrected by image registration (FLIRT, FSL). For this purpose the first echo images of the multi echo MGE and MSME acquisitions were used. These images were registered onto the baseline scan. The resulting spatial transformation matrices were applied to the corresponding parametric maps.

For quantitative analysis of T2* and T2 regions of interest (ROIs) were defined according to the morphological features of the kidney. For this purpose, the layers (cortex (C), outer medulla (OM), inner medulla (IM)) were identified and measured in a series of freshly extracted rat kidneys [18]. The layers' dimensions were related to the individual kidney's length and width in the coronal view. From these measurements, a standardized model was derived that comprises a rectangular frame that tightly encloses the kidney and predefined sizes and positions of the ROIs relative to this frame such that the ROIs were accurately located within the respective layer (Figure 11). The positions of nine ROIs were defined: three ROIs in C (C1-C3), three ROIs in OM (O1-O3), and three ROIs in IM (I1-I3) as illustrated in Figure 11. The mean size of the ROIs was: C1, C3, O1, O3: 17 pixel, C2, O2: 46 pixel, I1, I3: 25 pixel, I2: 70 pixel. The segmentation software limited the operator interaction to the placement of a rectangular reference frame around the kidney. All ROIs were placed in safe distance from the borders between these kidney layers to avoid any 'contamination' from the neighboring layers (partial segment effects) and to allow for inter-individual variations in morphology without the need to change the ROI position. The earlier described kidney segmentation model [18] was adapted for the current study to avoid overlap of the ROIs with the locations of the pO2/Laser-flux probes (Fig. 11). Mean T2* and T2 values were calculated over the three ROIs placed in each kidney layer. The size of the reference rectangle, which tightly encloses the kidney, was used as an estimate for kidney size.

The invasively measured physiological data were averaged over the time period of the corresponding MR scans with the exception of the Laser-flux signals. Here 5s data intervals starting 1 s after the end of the MR acquisitions were used. This approach helped to exclude MR induced artifacts in the Laser-flux signals [12].

### Statistical Analysis:

To test our hypothesis the relation between invasively measured physiological parameters and parameters derived from MRI was assessed. For this purpose relative changes in the physiological parameters and in kidney size were tested for correlation with relative changes in T2* and T2 for all renal layers and for the three interventions. Spearman's analysis (non-parametric correlation on ranks) was used, by which the strength of relationships is assessed that follow a monotonous function. If such a significant correlation was observed, additionally Pearson's analysis (parametric correlation) was applied, by which the strength and parameters of linear relationships are assessed. A p-value p<0.05 was considered to be statistically relevant.

### Results from Example 2:

### Animal Preparation:

Notwithstanding the experimental challenges dictated by the space constraints of the small bore MR scanner, surgical preparation was successfully performed in 15 out of 21 animals. The most frequent complication during surgery was unintended obstruction of renal blood flow caused by the vascular flow probe. To identify and exclude invalid data due to surgical, technical or physiological reasons all in vivo data were thoroughly examined. MR imaging scouts were used to check the positioning of the perivascular flow probe and the cortical and medullary Laser-flux/pO2 probes. All renal T2* maps were carefully checked for susceptibility artifacts induced by the surgical preparation or by the probes, which yielded data sets free of severe susceptibility artifacts for each renal layer for 15 animals. The invasively acquired physiological parameters were benchmarked against data and experience derived from our previous studies which included physiological measurements in large cohorts of animals [48-51].

### Renal T2* and T2:

Figure 12 shows exemplary T2* and T2 maps obtained during baseline, aortic occlusion, hypoxia, and hyperoxia together with ΔT2* and ΔT2 difference maps. The latter were determined by subtracting T2* and T2 maps acquired at the last time point of the intervention phase from baseline. At baseline the inner medulla including the papilla revealed T2*/T2 values that were markedly higher versus T2*/T2 observed for the cortex and outer medulla. Parametric maps showed a cortical intra-layer T2*/T2 pattern that indicates that the spatial resolution affords visualization of spatial variability in intra-layer pO2, which is related to the distance to the vascular bundles [52, 53].

Hyperoxia induced rather uniform increase in renal T2* and T2 across the kidney as outlined by exemplary maps in Figure 12. Averaged over all animals maximum T2* changes were 19±3% (C), 22±2% (OM), 7±4% (IM) and maximum T2 changes were 7±1 % (C), 9±1 % (OM), 8±2% (IM). Aortic occlusion caused a T2*/T2 decrease for all renal layers. Averaged over all animals maximum T2* changes were -11±2% (C), -42±2% (OM), -17±5% (IM) and maximum T2 changes were -17±3% (C), -22±3% (OM), -6±6% (IM). Hypoxia induced rather uniform reduction in renal T2* and T2 with maximum ΔT2* -47±3% (C), -59±2% (OM), and -37±5% (IM)), and maximum ΔT2 -28±3% (C), -36±4% (OM), and -20±6% (IM)).

### Time Course of Physiological and MR Parameters during Interventions:

Figure 13 outlines the time course of physiological and MR parameters monitored during hyperoxia & recovery. After onset of hyperoxia tissue pO2 started to increase and reached 240% over baseline in the cortex and 60% over baseline in the medulla. This pO2 increase was accompanied by small rises in RPP (7%) and RBF (8%). Laser-fluxes, renal conductance and kidney size remained largely unchanged. T2* increased versus baseline with maxima of 19% in the cortex, 22% in the outer medulla, and 7% in the inner medulla. T2 maps revealed smaller increases (C: 7%, OM: 9%, IM: 8%). After switching to normoxia tissue pO2 slowly returned towards baseline. T2* and T2 returned to baseline more rapidly. RBF and RPP even fell somewhat below baseline.

Figure 14 illustrates the time course obtained for physiological and MR parameters during aortic occlusion & recovery. With onset of occlusion RBF immediately ceased and kidney size decreased by 3.9%. Tissue pO2 rapidly decreased: within 90 seconds cortical pO2 had dropped by 90% and medullary pO2 by 97%. T2* became also reduced, but much less than pO2. T2* displayed a different time course among the layers. Outer medullary T2* declined instantly by 42% while cortical and inner medullary T2* decreased only modestly by 11% and 17%. After onset of reperfusion cortical and inner medullary T2* initially continued to decrease and reached a level of 71% of baseline in the cortex and 69% of baseline in the inner medulla before starting to re-increase. Excursions of T2 were even smaller than those of T2*. In response to the occluder's deflation RPP returned to baseline within 5 minutes while RBF's return to baseline was more slowly. Consequently renal conductance started significantly below baseline (not measurable during the occlusion) and slowly approximated baseline. Kidney size followed this trend and regained baseline within 8 minutes after reperfusion. Cortical and medullary pO2 followed the gradual recovery of RBF and reached baseline level only 8 minutes into reperfusion. While cortical and tissue pO2 started to improve immediately after onset of reperfusion, T2* initially remained unchanged (OM) or was even further reduced (C, IM) before restoration to baseline values began.

Figure 15 depicts the time course obtained for physiological and MR parameters in response to the hypoxia & recovery maneuver. With the onset of hypoxia RPP declined by 46% and RBF decreased even more (by 70%). Renal conductance dropped by 53% and kidney size by 5%.

Tissue pO2 gradually decreased: within 7 minutes of hypoxia medullary pO2 had decreased by 96% and cortical pO2 by 66%. T2* and T2 changes were found to be in sync with the pO2 alterations. The drop in T2* and T2 observed during hypoxia was markedly larger than during occlusion. During hypoxia T2* fell by 47% in C, 59% in OM, and 37% in IM. After switching to normoxia RPP, RBF, conductance and renal tissue pO2 started to recover immediately and returned to baseline. The tissue hypoxia to normoxia transition was faster versus aortic occlusion-recovery. T2* and pO2 time courses slightly deviated during recovery from hypoxia as cortical and medullary pO2 displayed some oscillations.

### Correlations of Physiological and MR Parameters:

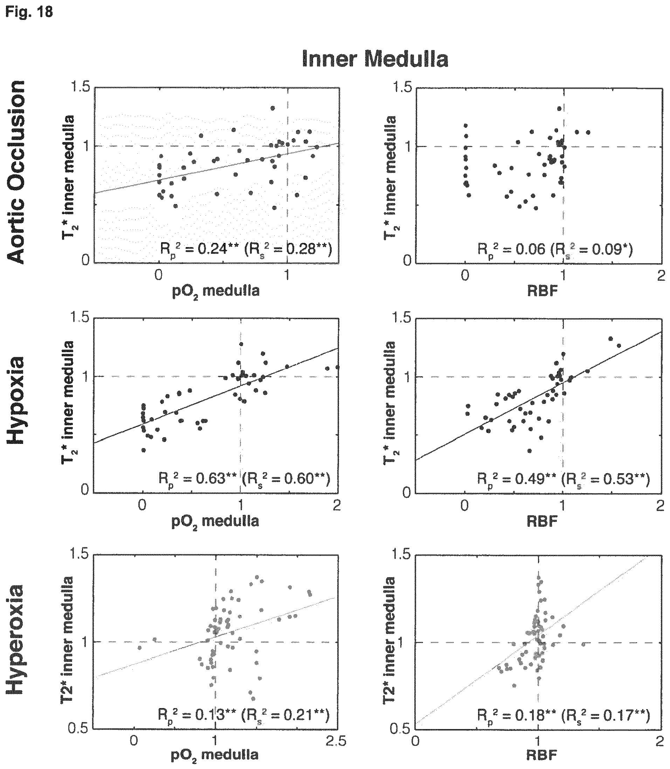

Table 1 provides a synopsis of the correlations between physiological parameters and MR parameters in response to hyperoxia & recovery, occlusion & recovery, and hypoxia & recovery. Significant Spearman rank correlations between T2* and tissue pO2 were observed for all interventions and all renal layers as illustrated in Figures 16, 17, and 18. The weakest T2*/pO2 correlation was found during hyperoxia & recovery, a closer one in response to occlusion & recovery, and the closest T2*/pO2 correlation during hypoxia & recovery (Table 1). The inter-layer comparison revealed weakest T2*/pO2 correlations for the cortex (Figure 16) and closest correlations for the outer medulla (Figure 17).

Pearson's analysis revealed significant linear correlations of T2* with outer medullary tissue pO2 for all interventions. Cortical T2* and pO2 showed a significant linear correlation during occlusion & recovery only. This is plausible since the average O2 saturation of Hb in most cortical vessels will be above 75% (i.e. in the non-linear range of the sigmoid oxyHb dissociation curve) under most conditions but not during the occlusion period, whereas saturation in medullary vessels will be lower (i.e., in the range of the curve that approaches linearity).

Significant Spearman correlations of T2* to RBF were deduced for all renal layers and for all interventions (Figures 16-18). The closest correlation was observed for the outer medulla followed by the cortex and the inner medulla. The T2*/RBF correlations for cortex and outer medulla were even superior to those between T2* and tissue pO2. The closest T2*/RBF correlation occurred during hypoxia & recovery.

T2*/RPP correlations were similar to T2*/RBF correlations as outlined in Table A. Surprisingly close correlations were observed between T2* and kidney size during hypoxia & recovery and for occlusion & recovery. In both cases, kidney size correlated well with renal vascular conductance (Table B), as did renal vascular conductance with T2* (Table A). Significant correlations of RBF to tissue pO2 were observed for all kidney layers during hypoxia & recovery and for occlusion & recovery (Table B). These correlations were superior to the T2*/pO2 correlations observed for occlusion & recovery and hypoxia & recovery.

### Discussion:

Our results demonstrate that the present setup is instrumental to detail the link between renal tissue pO2 and T2* in vivo. This is of essence to address the weakness of MRI, its qualitative nature, by benchmarking the surrogate MRI biomarkers against invasive methods while putting MRI's ability to non-invasively capture the physiological heterogeneity between and within the renal layers to good use. The technical challenges and practical obstacles of the MR environment were successfully offset. Remotely controlled standardized interventions were implemented in the MR scanner in order to systematically examine the validity and efficacy of parametric MRI as a surrogate marker for renal oxygenation. These efforts are of high relevance for research into the pathogenesis of renal diseases that are induced or promoted by renal tissue hypoperfusion and hypoxia.

Our findings indicate that changes in T2* qualitatively reflect changes in renal tissue pO2 induced by hyperoxia, aortic occlusion, and hypoxia. This is in alignment with the link between T2*, O2-saturation of Hb, blood pO2, and tissue pO2. A closer examination of the quantitative relation between relative changes in T2* and in tissue pO2 revealed discrepancies that point at factors other than the known shifts of the deoxyHb dissociation curve and changes in haematocrit, which may also confound the renal T2*/ tissue pO2 relationship. Major differences in the T2*/pO2 correlations together with the stark differences in the linear regression of T2*/pO2 indicate that simple translation of quantitative results obtained for one intervention of renal hemodynamics and oxygenation to another intervention is falling short from being appropriate. Also, taking the perfusion and oxygenation heterogeneity within any given kidney layer into account, extrapolation of results obtained for specific renal regions to other renal areas must be made with due caution.

Hyperoxia & recovery yielded weak correlations for tissue pO2 changes versus T2* changes for all kidney layers. This observation is plausible since almost all of the available Hb in arterial blood is already O2 saturated under normoxic conditions. Increasing the inspiratory oxygen fraction to 100% barely lifts the O2 saturation of Hb in arterial blood. It does substantially increase arterial blood pO2 though, which enhances the driving force for O2 diffusion from vessels to tissue so that renal tissue pO2 increases dramatically. The difference in the pO2 increase between cortex and medulla is likely due to arterio-venous diffusive O2 shunting, which reduces the oxygen content of arterial blood that perfuses the medulla [3, 38, 52]. Benchmarked against the massive pO2 increase T2* changes were much smaller. Hyperoxia-induced increase of arterial blood oxygen content are barely detectable by T2*- mapping since the deoxyHb concentration is virtually unaffected. However, hyperoxia increases blood pO2 in intrarenal veins due to increasing arterio-venous oxygen shunting in case of a higher arterio-venous pO2 difference [3, 38]. As renal venous Hb is far from being completely saturated with O2 under normoxic conditions hyperoxia induced increase in venous blood pO2 translates into the small increase in T2* observed in our study.

Aortic occlusion & recovery showed closer tissue pO2/T2* correlations for all renal layers versus those observed in response to hyperoxia & recovery. Occlusion of the suprarenal aorta results in abrupt cessation of blood flow into the kidney while renal O2 consumption remains unaltered at this early stage. This leads to a rapid and massive decline in renal tissue pO2, which reduces blood pO2 and O2 saturation of Hb in the intrarenal (micro-)vasculature. This intrarenal Hb deoxygenation is aggravated by a progressive rightward shift of the oxyHb dissociation curve during the occlusion due to intrarenal accumulation of CO2. The increase in deoxyHb is reflected by the T2* decrease observed during occlusion for all layers. The T2* decrease was mild and slow as compared to the decline in pO2 though, and displayed different characteristics for the kidney layers. These findings can be attributed to blood volume fraction changes which occurred during occlusion. While blood flow into kidney is abruptly stopped by the occlusion, outflow of blood via the renal vein will continue until pressures in intrarenal vessels and in the vena cava are equalized. This results in a reduction of intrarenal blood volume [54] and manifests itself in the immediate drop in kidney size shown here. Since the total volume of the other renal fluid compartments including the tubular, interstitial, and cellular fraction is probably largely unchanged, the blood volume fraction becomes markedly reduced. As T2* is linked to the volume fraction of deoxyHb, the reduction in blood volume fraction compensates some of the increased deoxyHb blood concentration induced changes in T2*. Consequently, the T2* decrease turns out to be smaller than the actual change in blood (and tissue) oxygenation. It is plausible that this effect is much more pronounced in the cortex than in the medulla, due to the much larger blood volume in the cortex [4, 38]. T2* in the cortex follows the declining blood (tissue) pO2 less closely than in the outer medulla. The inner medulla's metabolism is largely anaerobic [55], hence the remaining oxygen in the stationary blood is sufficient for a longer time span so that pO2 and T2* decline only slowly.

By deflating the aortic occluder the kidney is reperfused. Complete restoration of RBF took about 8 minutes so that oxygen delivery recovers only gradually. At the same time, glomerular filtration, which was arrested during occlusion, is gradually restored. The ensuing restoration of tubular reabsorption necessitates higher O2 consumption. Limited oxygen delivery and increased demand result in the observed slow recovery of tissue pO2. The substantial O2 extraction from the freshly inflowing blood is embodied by a low T2*. Although tissue pO2 started to improve immediately after onset of reperfusion, cortical T2* continued to decline even further before it started to recover. Again, this discrepancy most probably relies on a change in the blood volume fraction. With reperfusion renal blood volume increases while the total volume of the other fluid compartments is largely unchanged. Increased blood volume fraction at the onset of reperfusion leads T2* to an overestimation of tissue hypoxia. The increase in blood volume is somewhat attenuated by renal vasoconstriction triggered by renal autoregulatory mechanisms [56], as evident from renal vascular conductance being significantly below baseline right after the onset of reperfusion. The attenuated recovery of intrarenal blood volume is also mirrored by the somewhat delayed restoration in kidney size.

Hypoxia & recovery revealed the strongest renal tissue pO2/T2* correlation for all interventions applied. Switching the inspiratory oxygen fraction to 8% resulted in arterial hypoxemia so that tissue pO2 gradually decreased and reached a steady state. The major reason behind the pronounced decrease in medullary pO2 is that the medulla is predominantly perfused by blood that had already traversed the cortex, where blood oxygen content is lowered by oxygen extraction and arterio-venous oxygen shunt diffusion [3, 4, 38, 52]. As expected, the pO2 decrease during hypoxia was slower and with regard to the cortex also less pronounced than during aortic occlusion. In contrast, the T2* response to hypoxia was significantly more pronounced versus aortic occlusion. It is evident that aortic occlusion must lower intrarenal blood oxygenation much more and faster than hypoxia. In addition, intrarenal pCO2 increases during occlusion due to CO2 accumulation, but decreases during hypoxia due to hyperventilation triggered by systemic hypoxemia [57]. Decreased pCO2 shifts the deoxyHb dissociation curve to the left so that at given pO2 the O2 saturation of Hb is larger. Increased pCO2 induces opposite effects. For all these reasons, T2* decrease should be more pronounced during aortic occlusion versus hypoxia. Our results indicate that the T2* response to FiO2 8% overestimates the actual degree of blood (and tissue) hypoxia. The decrease in renal conductance during hypoxia suggests that renal vasoconstriction lowered intrarenal blood volume. Yet the vasoconstriction-related drop in renal blood volume during hypoxia is most likely smaller versus the outflow-induced drop during aortic occlusion. Intriguingly, the decrease in kidney size was somewhat larger during hypoxia than during occlusion. This observation points at an additional volume loss of renal tissue compartments other than blood. Contrary to aortic occlusion, where glomerular filtration ceases and with it the pressure gradient that drives tubular fluid toward the renal pelvis, filtration and tubular fluid flow will decrease but not cease during hypoxia. The outflow lowers tubular volume. Moreover, reabsorbed fluid cannot be drained by peritubular capillaries during occlusion because of the arrested blood flow such that the sum of the tubular plus interstitial volumes remains constant. As peritubular capillary blood flow is not arrested during hypoxia, reabsorbed fluid is continued to be drained from the interstitium. Since the decrease in tubular volume is not counterbalanced by an increase in the interstitial volume, the sum of both volumes decreases. This translates into a larger decrease in the blood volume fraction during occlusion versus hypoxia which might explain the discrepancies in the T2* and pO2 response to hypoxia and aortic occlusion.

Significant rank correlations of RBF to T2* were observed for all renal layers and for all interventions. These correlations were closer than those of tissue pO2 to T2* for the cortex and outer medulla. Tissue pO2 primarily reflects the balance between O2 supply and O2 demand. Due to O2 shunt diffusion, blood pO2 in larger arterial and venous vessels exceeds that in capillaries and tissue pO2. The amount of O2 that is shunted depends on O2 consumption but also on RBF and arterial O2 content [3, 4, 38, 52]. In the cortex, the effect of changes in RBF on shunting appears to be enhanced: the correlations of RBF to cortical tissue pO2 were much weaker than those of RBF and medullary tissue pO2. When comparing RBF/T2* versus pO2/T2* it should be taken into account that the pO2 probes 120 µm) subjacent to the probes' tip [2, □cover a rather small tissue volume (r 12]. The individual position of a given probe in relation to larger vessels versus capillaries will therefore determine the individual absolute pO2 values, and may also impinge on the relative changes during interventions. Unlike pO2 measurements RBF measurements are unaffected by intrarenal spatial variability.

To summarize, this work presents valuable insights into the mechanisms behind alterations in renal T2* by detailing the link between renal T2* and renal tissue pO2. Yet, a singular report eloquently refers to simultaneous measurements of renal R2* (R2* = 1/T2*) and tissue pO2 [47]. The authors modulated FiO2 (range: 5-70%) in pigs and reported R2*changes to be linearly related with pO2 changes. This conclusion appears somewhat premature, since T2* was measured in the contralateral kidney with the pO2 probe being placed in the ipsilateral kidney. It should be also noted that correlation analysis was based upon group means rather than individual data pairs as used here.

The overall qualitative agreement of T2* and pO2 changes observed in the present study encourages further research into calibration of renal T2* alterations using MR-PHYSIOL. Notwithstanding this success, our findings generated novel questions about the renal T2*/tissue pO2 relation. It stands to reason that T2* is directly related to the amount of deoxyHb per tissue volume and hence linked with tissue pO2 via blood pO2 and the oxyHb dissociation curve. However, the T2* to tissue pO2 correlation differences between the interventions and our renal vascular conductance and kidney size data indicate that changes in the blood volume fraction considerably influence renal T2*. Renal vascular conductance changes point at changes in intrarenal blood volume that occur by passive circular distension induced by changes in the transmural pressure gradient, or by active vasomotion. Changes in kidney size may stem from volume changes in any of the renal fluid compartments. There are at least two compartments besides the vascular one that can experience rapid volume changes, which in turn modulate the blood volume fraction: the interstitial and the tubular compartment, with the latter being a particularity of the kidney. The tubular volume fraction is quite large and can rapidly change due (i) changes in filtration, (ii) alterations in tubular outflow towards the pelvis, (iii) modulation of the transmural pressure gradient, and (iv) changes in resorption. A recent report recognized that changes in blood volume fraction induced by changes in tubular volume may impact renal T2* [17] by showing that the renal T2* response indicated an increased oxygenation immediately after x-ray contrast agent administration rather than decreased oxygenation [17]. The dependence of R2=1/T2 on alterations in tubular volume has also been observed upon administration of vasoactive substances [58].

Unravelling the link between regional renal T2* and tissue pO2 - including the role of the T2* confounding parameters vascular and tubular volume fraction and oxyHb dissociation curve - requires further research. Blood volume fraction, tubular volume fraction and oxyHb dissociation curve T2* contributions must be differentiated from renal BOLD T2* changes in order to provide quantitative means for interpretation of renal hemodynamics/oxygenation. These efforts should make use of the advanced capabilities of the present system by including parallel imaging techniques to improve the temporal resolution [59-62], by investing into dual contrast techniques for simultaneous T2*/T2 weighted MRI [63], by driving T2* mapping techniques free of image distortion [64] but also MR based assessment of renal blood volume [65] and by probing tubular volume fraction using diffusion weighted or intra-voxel incoherent motion techniques [43, 66-69], while blood sampling may be employed to examine the role of shifts in the oxyHb dissociation curve. These explorations are essential before the quantitative capabilities of parametric MRI can be translated from experimental research to improved clinical understanding of hemodynamics/oxygenation in kidney disorders.

### References for Example 2:

1. Singh P, Ricksten SE, Bragadottir G, et al. Renal oxygenation and haemodynamics in acute kidney injury and chronic kidney disease. Clin Exp Pharmacol Physiol. 2013;40:138-47.
2. Evans RG, Gardiner BS, Smith DW, et al. Methods for studying the physiology of kidney oxygenation. Clin Exp Pharmacol Physiol. 2008;35:1405-12.
3. Evans RG, Goddard D, Eppel GA, et al. Factors that render the kidney susceptible to tissue hypoxia in hypoxemia. Am J Physiol Regul Integr Comp Physiol. 2011;300:R931-40.
4. Evans RG, Ince C, Joles JA, et al. Haemodynamic influences on kidney oxygenation: clinical implications of integrative physiology. Clin Exp Pharmacol Physiol. 2013;40:106-22.
5. Chawla LS, Kimmel PL. Acute kidney injury and chronic kidney disease: an integrated clinical syndrome. Kidney Int. 2012;82:516-24.
6. Seeliger E, Sendeski M, Rihal CS, et al. Contrast-induced kidney injury: mechanisms, risk factors, and prevention. Eur Heart J. 2012;33:2007-15.
7. Legrand M, Mik EG, Johannes T, et al. Renal hypoxia and dysoxia after reperfusion of the ischemic kidney. Mol Med. 2008;14:502-16.
8. Friederich-Persson M, Thorn E, Hansell P, et al. Kidney hypoxia, attributable to increased oxygen consumption, induces nephropathy independently of hyperglycemia and oxidative stress. Hypertension. 2013;62:914-9.
9. Hansell P, Welch WJ, Blantz RC, et al. Determinants of kidney oxygen consumption and their relationship to tissue oxygen tension in diabetes and hypertension. Clin Exp Pharmacol Physiol. 2013;40:123-37.
10. Prasad PV. Functional MRI of the kidney: tools for translational studies of pathophysiology of renal disease. Am J Physiol Renal Physiol. 2006;290:F958-74.
11. Evans RG, Leong CL, Anderson WP, et al. Don't be so BOLD: potential limitations in the use of BOLD MRI for studies of renal oxygenation. Kidney Int. 2007;71:1327-8; author reply 8.
12. Pohlmann A, Cantow K, Hentschel J, et al. Linking non-invasive parametric MRI with invasive physiological measurements (MR-PHYSIOL): towards a hybrid and integrated approach for investigation of acute kidney injury in rats. Acta Physiol (Oxf). 2013;207:673-89.
13. Prasad PV, Epstein FH. Changes in renal medullary pO2 during water diuresis as evaluated by blood oxygenation level-dependent magnetic resonance imaging: effects of aging and cyclooxygenase inhibition. Kidney Int. 1999;55:294-8.
14. Prasad PV, Priatna A, Spokes K, et al. Changes in intrarenal oxygenation as evaluated by BOLD MRI in a rat kidney model for radiocontrast nephropathy. J Magn Reson Imaging. 2001;13:744-7.
15. Li LP, Halter S, Prasad PV. Blood oxygen level-dependent MR imaging of the kidneys. Magn Reson Imaging Clin N Am. 2008;16:613-25, viii.
16. Haque M, Franklin T, Prasad P. Renal oxygenation changes during water loading as evaluated by BOLD MRI: effect of NOS inhibition. J Magn Reson Imaging. 2011;33:898-901.
17. Arakelyan K, Cantow K, Hentschel J, et al. Early effects of an x-ray contrast medium on renal T(2) */T(2) MRI as compared to short-term hyperoxia, hypoxia and aortic occlusion in rats. Acta Physiol (Oxf). 2013;208:202-13.
18. Pohlmann A, Hentschel J, Fechner M, et al. High temporal resolution parametric MRI monitoring of the initial ischemia/reperfusion phase in experimental acute kidney injury. PLoS One. 2013;8:e57411.
19. Peng XG, Bai YY, Fang F, et al. Renal Lipids and Oxygenation in Diabetic Mice: Noninvasive Quantification with MR Imaging. Radiology. 2013;269:748-57.
20. Morrell GR, Zhang JL, Lee VS. Science to Practice: Renal Hypoxia and Fat Deposition in Diabetic Neuropathy-New Insights with Functional Renal MR Imaging. Radiology. 2013;269:625-6.
21. Menzies RI, Zammit-Mangion A, Hollis LM, et al. An anatomically unbiased approach for analysis of renal BOLD magnetic resonance images. Am J Physiol Renal Physiol. 2013;305:F845-52.
22. Pruijm M, Hofmann L, Charollais-Thoenig J, et al. Effect of dark chocolate on renal tissue oxygenation as measured by BOLD-MRI in healthy volunteers. Clin Nephrol. 2013;80:211-7.
23. Pruijm M, Hofmann L, Vogt B, et al. Renal tissue oxygenation in essential hypertension and chronic kidney disease. Int J Hypertens. 2013;2013:696598.
24. Pruijm M, Hofmann L, Zanchi A, et al. Blockade of the renin-angiotensin system and renal tissue oxygenation as measured with BOLD-MRI in patients with type 2 diabetes. Diabetes Res Clin Pract. 2013;99:136-44.
25. Liss P, Cox EF, Eckerbom P, et al. Imaging of intrarenal haemodynamics and oxygen metabolism. Clin Exp Pharmacol Physiol. 2013;40:158-67.
26. Gloviczki ML, Lerman LO, Textor SC. Blood oxygen level-dependent (BOLD) MRI in renovascular hypertension. Curr Hypertens Rep. 2011;13:370-7.
27. Rognant N, Lemoine S, Laville M, et al. [Evaluation of renal oxygen content by BOLD MRI]. Nephrol Ther. 2012;8:212-5.
28. Park SY, Kim CK, Park BK, et al. Evaluation of transplanted kidneys using blood oxygenation level-dependent MRI at 3 T: a preliminary study. AJR Am J Roentgenol. 2012;198:1108-14.
29. Donati OF, Nanz D, Serra AL, et al. Quantitative BOLD response of the renal medulla to hyperoxic challenge at 1.5 T and 3.0 T. NMR Biomed. 2012;25:1133-8.
30. Ebrahimi B, Gloviczki M, Woollard JR, et al. Compartmental analysis of renal BOLD MRI data: introduction and validation. Invest Radiol. 2012;47:175-82.
31. Chrysochou C, Mendichovszky IA, Buckley DL, et al. BOLD imaging: a potential predictive biomarker of renal functional outcome following revascularization in atheromatous renovascular disease. Nephrol Dial Transplant. 2012;27:1013-9.
32. Rognant N, Guebre-Egziabher F, Bacchetta J, et al. Evolution of renal oxygen content measured by BOLD MRI downstream a chronic renal artery stenosis. Nephrol Dial Transplant. 2011;26:1205-10.
33. Warner L, Glockner JF, Woollard J, et al. Determinations of renal cortical and medullary oxygenation using blood oxygen level-dependent magnetic resonance imaging and selective diuretics. Invest Radiol. 2011;46:41-7.
34. Gloviczki ML, Glockner J, Gomez SI, et al. Comparison of 1.5 and 3 T BOLD MR to study oxygenation of kidney cortex and medulla in human renovascular disease. Invest Radiol. 2009;44:566-71.
35. Rossi C, Sharma P, Pazahr S, et al. Blood oxygen level-dependent magnetic resonance imaging of the kidneys: influence of spatial resolution on the apparent R2* transverse relaxation rate of renal tissue. Invest Radiol. 2013;48:671-7.
36. Ogawa S. Finding the BOLD effect in brain images. Neuroimage. 2012;62:608-9.
37. Griffeth VE, Buxton RB. A theoretical framework for estimating cerebral oxygen metabolism changes using the calibrated-BOLD method: modeling the effects of blood volume distribution, hematocrit, oxygen extraction fraction, and tissue signal properties on the BOLD signal. Neuroimage. 2011;58:198-212.
38. Evans RG, Gardiner BS, Smith DW, et al. Intrarenal oxygenation: unique challenges and the biophysical basis of homeostasis. Am J Physiol Renal Physiol. 2008;295:F1259-70.
39. Michaely HJ, Metzger L, Haneder S, et al. Renal BOLD-MRI does not reflect renal function in chronic kidney disease. Kidney Int. 2012;81:684-9.
40. Fine LG, Dharmakumar R. Limitations of BOLD-MRI for assessment of hypoxia in chronically diseased human kidneys. Kidney Int. 2012;82:934-5; author reply 5.
41. Inoue T, Kozawa E, Okada H, et al. Is there no future for renal BOLD-MRI? Kidney Int. 2012;82:934; author reply 5.
42. Dean DA, Jia CX, Cabreriza SE, et al. Validation study of a new transit time ultrasonic flow probe for continuous great vessel measurements. ASAIO journal (American Society for Artificial Internal Organs : 1992). 1996;42:M671-6.
43. Hueper K, Rong S, Gutberlet M, et al. T2 relaxation time and apparent diffusion coefficient for noninvasive assessment of renal pathology after acute kidney injury in mice: comparison with histopathology. Invest Radiol. 2013;48:834-42.
44. Juillard L, Lerman LO, Kruger DG, et al. Blood oxygen level-dependent measurement of acute intra-renal ischemia. Kidney Int. 2004;65:944-50.
45. dos Santos EA, Li L-P, Ji L, et al. Early changes with diabetes in renal medullary hemodynamics as evaluated by fiberoptic probes and BOLD magnetic resonance imaging. Investigative radiology. 2007;42:157-62.
46. Li L-P, Ji L, Santos EA, et al. Effect of nitric oxide synthase inhibition on intrarenal oxygenation as evaluated by blood oxygenation level-dependent magnetic resonance imaging. Investigative radiology. 2009;44:67-73.
47. Pedersen M, Dissing T, Morkenborg J, et al. Validation of quantitative BOLD MRI measurements in kidney: Application to unilateral ureteral obstruction. Kidney Int. 2005;67:2305-12.
48. Flemming B, Seeliger E, Wronski T, et al. Oxygen and renal hemodynamics in the conscious rat. J Am Soc Nephrol. 2000;11:18-24.
49. Hoff U, Lukitsch I, Chaykovska L, et al. Inhibition of 20-HETE synthesis and action protects the kidney from ischemia/reperfusion injury. Kidney Int. 2011;79:57-65.
50. Seeliger E, Flemming B, Wronski T, et al. Viscosity of contrast media perturbs renal hemodynamics. J Am Soc Nephrol. 2007;18:2912-20.
51. Seeliger E, Cantow K, Arakelyan K, et al. Low-Dose Nitrite Alleviates Early Effects of an X-ray Contrast Medium on Renal Hemodynamics and Oxygenation in Rats. Invest Radiol. 2013. [epub ahead]
52. Schurek HJ. [Kidney medullary hypoxia: a key to understanding acute renal failure?]. Klin Wochenschr. 1988;66:828-35.
53. Lubbers DW, Baumgartl H. Heterogeneities and profiles of oxygen pressure in brain and kidney as examples of the pO2 distribution in the living tissue. Kidney Int. 1997;51:372- 80.
54. Grosenick D, Steinkellner O, Wabnitz H, et al. Near-infrared spectroscopy of renal tissue in vivo. Proc of SPIE. 2013.
55. Edwards A, Silldforff EP, Pallone TL. The renal medullary microcirculation. Front Biosci. 2000;5:E36-52.
56. Seeliger E, Wronski T, Ladwig M, et al. The renin-angiotensin system and the third mechanism of renal blood flow autoregulation. Am J Physiol Renal Physiol. 2009;296:F1334- 45.
57. Marshall JM, Metcalfe JD. Influences on the cardiovascular response to graded levels of systemic hypoxia of the accompanying hypocapnia in the rat. J Physiol. 1989;410:381-94.
58. Storey P, Ji L, Li LP, et al. Sensitivity of USPIO-enhanced R2 imaging to dynamic blood volume changes in the rat kidney. J Magn Reson Imaging. 2011;33:1091-9.
59. Niendorf T, Sodickson DK. Highly accelerated cardiovascular MR imaging using many channel technology: concepts and clinical applications. Eur Radiol. 2008;18:87-102.
60. Niendorf T, Sodickson DK. Parallel imaging in cardiovascular MRI: methods and applications. NMR Biomed. 2006;19:325-41.
61. Niendorf T, Saranathan M, Lingamneni A, et al. Short breath-hold, volumetric coronary MR angiography employing steady-state free precession in conjunction with parallel imaging. Magn Reson Med. 2005;53:885-94.
62. Niendorf T, Sodickson D. [Acceleration of cardiovascular MRI using parallel imaging: basic principles, practical considerations, clinical applications and future directions]. Rofo. 2006; 178: 15-30.
63. Fuchs K, Hezel F, Klix S, et al. Simultaneous dual contrast weighting using double echo rapid acquisition with relaxation enhancement (RARE) imaging. Magn Reson Med. 2013.[epub ahead]
64. Heinrichs U, Utting JF, Frauenrath T, et al. Myocardial T2* mapping free of distortion using susceptibility-weighted fast spin-echo imaging: a feasibility study at 1.5 T and 3.0 T. Magn Reson Med. 2009;62:822-8.
65. Wang F, Jiang RT, Tantawy MN, et al. Repeatability and sensitivity of high resolution blood volume mapping in mouse kidney disease. J Magn Reson Imaging. 2013.
66. Eckerbom P, Hansell P, Bjerner T, et al. Intravoxel incoherent motion MR imaging of the kidney: pilot study. Adv Exp Med Biol. 2013;765:55-8.
67. Zhang JL, Sigmund EE, Rusinek H, et al. Optimization of b-value sampling for diffusion-weighted imaging of the kidney. Magn Reson Med. 2012;67:89-97.
68. Ichikawa S, Motosugi U, Ichikawa T, et al. Intravoxel incoherent motion imaging of the kidney: alterations in diffusion and perfusion in patients with renal dysfunction. Magn Reson Imaging. 2013;31:414-7.
69. Niendorf T, Dijkhuizen RM, Norris DG, et al. Biexponential diffusion attenuation in various states of brain tissue: implications for diffusion-weighted imaging. Magn Reson Med. 1996;36:847-57.

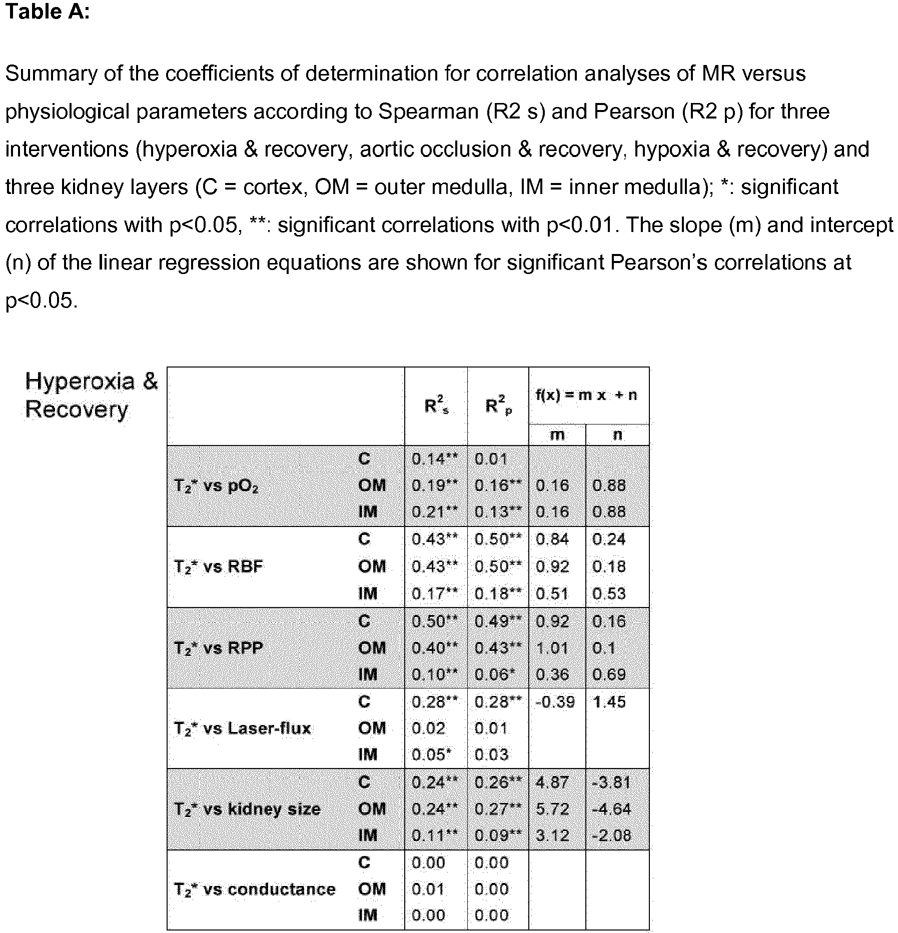

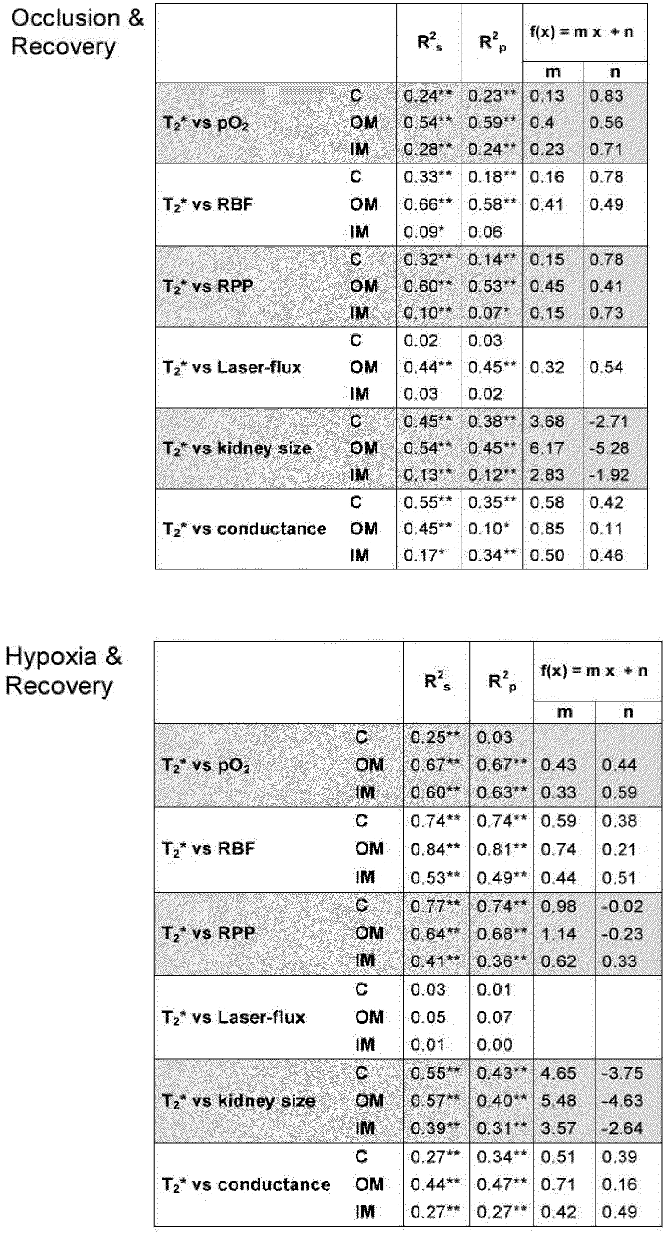

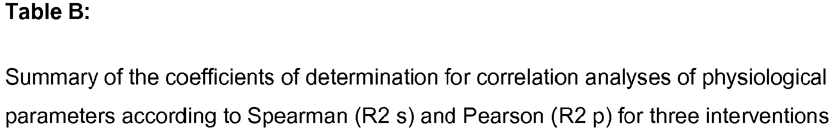

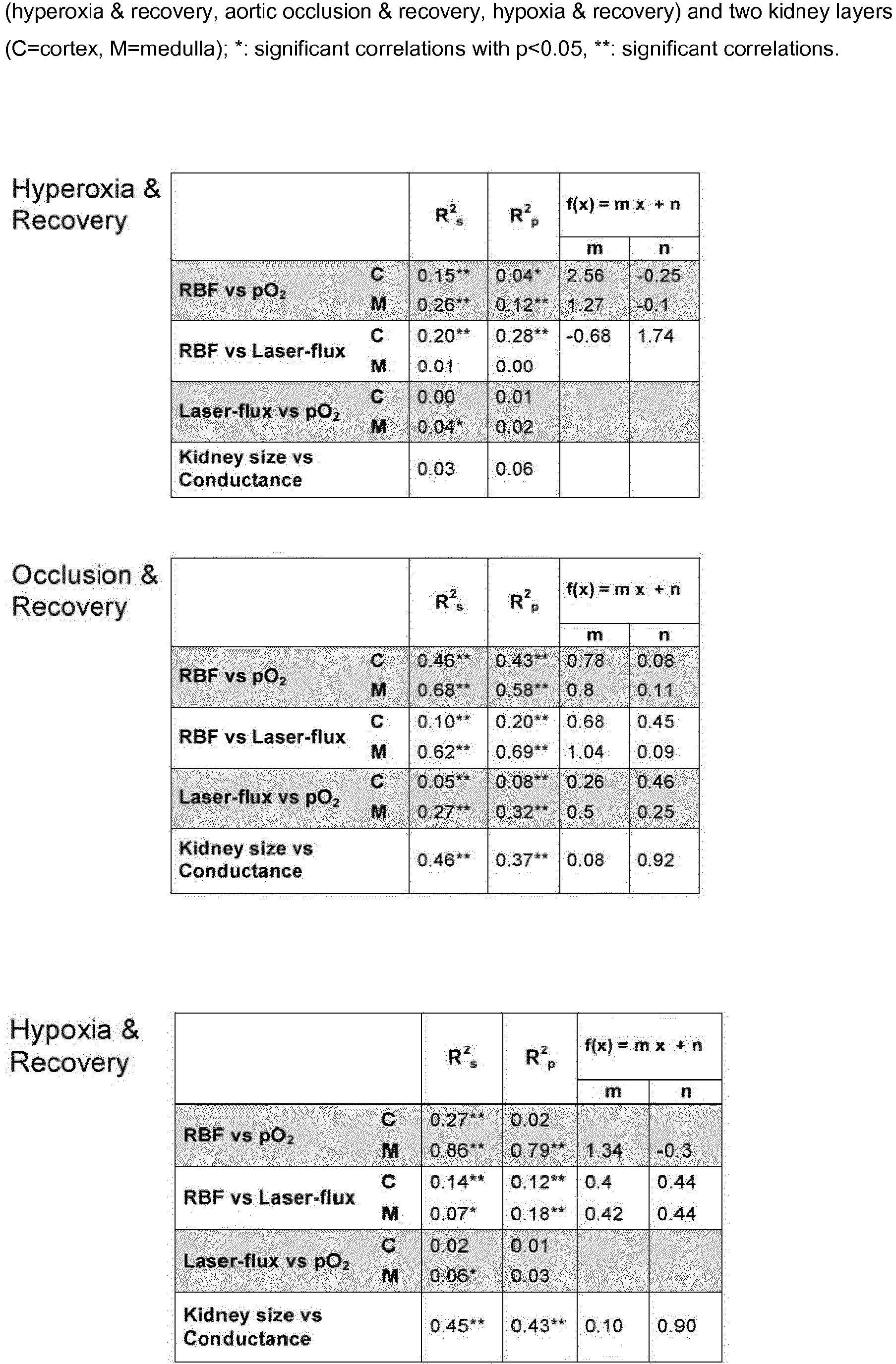

### References

Ahmeda, A. F. & Johns, E. J. 2012. The regulation of blood perfusion in the renal cortex and medulla by reactive oxygen species and nitric oxide in the anaesthetised rat. Acta Physiol (Oxf), 204, 443-50.
Aksu, U., Demirci, C. & Ince, C. 2011. The pathogenesis of acute kidney injury and the toxic triangle of oxygen, reactive oxygen species and nitric oxide. Contrib Nephrol, 174, 119-28.
Alford, S. K., Sadowski, E. A., Unal, O., Polzin, J. A., Consigny, D. W., Korosec, F. R. & Grist, T. M. 2005. Detection of acute renal ischemia in swine using blood oxygen level-dependent magnetic resonance imaging. J Magn Reson Imaging, 22, 347-53.
Badzynska, B. & Sadowski, J. 2009. Differential action of bradykinin on intrarenal regional perfusion in the rat: waning effect in the cortex and major impact in the medulla. J Physiol, 587, 3943-53.
Badzynska, B. & Sadowski, J. 2012. Experimental selective elevation of renal medullary blood flow in hypertensive rats: evidence against short-term hypotensive effect. Acta Physiol (Oxf), 205, 484-93.
Baudelet, C. & Gallez, B. 2002. How does blood oxygen level-dependent (BOLD) contrast correlate with oxygen partial pressure (pO2) inside tumors? Magn Reson Med, 48, 980-6.
Baumgartl, H., Leichtweiss, H. P., Lubbers, D. W., Weiss, C. & Huland, H. 1972. The oxygen supply of the dog kidney: measurements of intrarenal pO 2. Microvasc Res, 4, 247-57.
Bellomo, R., Kellum, J. A. & Ronco, C. 2012. Acute kidney injury. Lancet, 380, 756-66.
Blantz, R. C. & Deng, A. 2007. Coordination of kidney filtration and tubular reabsorption: considerations on the regulation of metabolic demand for tubular reabsorption. Acta Physiol Hung, 94, 83-94.
Blantz, R. C., Deng, A., Miracle, C. M. & Thomson, S. C. 2007. Regulation of kidney function and metabolism: a question of supply and demand. Trans Am Clin Climatol Assoc, 118, 23-43.
Blantz, R. C. & Weir, M. R. 2004. Are the oxygen costs of kidney function highly regulated? Curr Opin Nephrol Hypertens, 13, 67-71.
Bonventre, J. V. & Yang, L. 2011. Cellular pathophysiology of ischemic acute kidney injury. J Clin Invest, 121, 4210-21.
Bougle, A. & Duranteau, J. 2011. Pathophysiology of sepsis-induced acute kidney injury: the role of global renal blood flow and renal vascular resistance. Contrib Nephrol, 174, 89-97.
Brezis, M., Heyman, S. N. & Epstein, F. H. 1994. Determinants of intrarenal oxygenation. II. Hemodynamic effects. Am J Physiol, 267, F1063-8.
Brezis, M. & Rosen, S. 1995. Hypoxia of the renal medulla--its implications for disease. N Engl J Med, 332, 647-55.
Dantzler, W. H., Pannabecker, T. L., Layton, A. T. & Layton, H. E. 2011. Urine concentrating mechanism in the inner medulla of the mammalian kidney: role of three-dimensional architecture. Acta Physiol (Oxf), 202, 361-78.
Dean, D. A., Jia, C. X., Cabreriza, S. E., D'Alessandro, D. A., Dickstein, M. L., Sardo, M. J., Chalik, N. & Spotnitz, H. M. 1996. Validation study of a new transit time ultrasonic flow probe for continuous great vessel measurements. ASAIOJ, 42, M671-6.
Dobrowolski, L. & Sadowski, J. 2005. Furosemide-induced renal medullary hypoperfusion in the rat: role of tissue tonicity, prostaglandins and angiotensin II. J Physiol, 567, 613- 20.
Donahue, M. J., Hoogduin, H., van Zijl, P. C., Jezzard, P., Luijten, P. R. & Hendrikse, J. 2011. Blood oxygenation level-dependent (BOLD) total and extravascular signal changes and DeltaR2* in human visual cortex at 1.5, 3.0 and 7.0 T. NMR Biomed, 24, 25-34.
dos Santos, E. A., Li, L. P., Ji, L. & Prasad, P. V. 2007. Early changes with diabetes in renal medullary hemodynamics as evaluated by fiberoptic probes and BOLD magnetic resonance imaging. Invest Radiol, 42, 157-62.
Edlund, J., Hansell, P., Fasching, A., Liss, P., Weis, J., Glickson, J. D. & Palm, F. 2009. Reduced oxygenation in diabetic rat kidneys measured by T2* weighted magnetic resonance micro-imaging. Adv Exp Med Biol, 645, 199-204.
Edwards, A., Silldforff, E. P. & Pallone, T. L. 2000. The renal medullary microcirculation. Front Biosci, 5, E36-52. Epstein, F. H., Veves, A. & Prasad, P. V. 2002. Effect of diabetes on renal medullary oxygenation during water diuresis. Diabetes Care, 25, 575-8.
Evans, R. G., Bergstrom, G. & Lawrence, A. J. 1998. Effects of the vasopressin V1 agonist [Phe2,Ile3,Orn8]] vasopressin on regional kidney perfusion and renal excretory function in anesthetized rabbits. J Cardiovasc Pharmacol, 32, 571-81.
Evans, R. G., Eppel, G. A., Michaels, S., Burke, S. L., Nematbakhsh, M., Head, G. A., Carroll, J. F. & O'Connor, P. M. 2010. Multiple mechanisms act to maintain kidney oxygenation during renal ischemia in anesthetized rabbits. Am J Physiol Renal Physiol, 298, F1235-43.
Evans, R. G., Gardiner, B. S., Smith, D. W. & O'Connor, P. M. 2008a. Intrarenal oxygenation: unique challenges and the biophysical basis of homeostasis. Am J Physiol Renal Physiol, 295, F1259-70.
Evans, R. G., Gardiner, B. S., Smith, D. W. & O'Connor, P. M. 2008b. Methods for studying the physiology of kidney oxygenation. Clin Exp Pharmacol Physiol, 35, 1405-12.
Evans, R. G., Goddard, D., Eppel, G. A. & O'Connor, P. M. 2011a. Factors that render the kidney susceptible to tissue hypoxia in hypoxemia. Am J Physiol Regul Integr Comp Physiol, 300, R931-40.
Evans, R. G., Goddard, D., Eppel, G. A. & O'Connor, P. M. 2011b. Stability of tissue P02 in the face of altered perfusion: a phenomenon specific to the renal cortex and independent of resting renal oxygen consumption. Clin Exp Pharmacol Physiol, 38, 247-54.
Evans, R. G., Leong, C. L., Anderson, W. P. & O'Connor, P. M. 2007. Don't be so BOLD: potential limitations in the use of BOLD MRI for studies of renal oxygenation. Kidney Int, 71, 1327-8; author reply 1328.
Evans, R. G., Madden, A. C. & Denton, K. M. 2000. Diversity of responses of renal cortical and medullary blood flow to vasoconstrictors in conscious rabbits. Acta Physiol Scand, 169, 297-308. Flemming, B., Seeliger, E., Wronski, T., Steer, K., Arenz, N. & Persson, P. B. 2000. Oxygen and renal hemodynamics in the conscious rat. J Am Soc Nephrol, 11, 18-24.
Gardiner, B. S., Thompson, S. L., Ngo, J. P., Smith, D. W., Abdelkader, A., Broughton, B. R., Bertram, J. F. & Evans, R. G. 2012. Diffusive oxygen shunting between vessels in the preglomerular renal vasculature: anatomic observations and computational modeling. Am J Physiol Renal Physiol, 303, F605-18.
Gomez, S. I., Warner, L., Haas, J. A., Bolterman, R. J., Textor, S. C., Lerman, L. O. & Romero, J. C. 2009. Increased hypoxia and reduced renal tubular response to furosemide detected by BOLD magnetic resonance imaging in swine renovascular hypertension. Am J Physiol Renal Physiol, 297, F981-6.
Haneder, S., Augustin, J., Jost, G., Pietsch, H., Lengsfeld, P., Kramer, B. K., Schoenberg, S. O., Meyer, M., Attenberger, U. I. & Michaely, H. J. 2012. Impact of iso- and low- osmolar iodinated contrast agents on BOLD and diffusion MRI in swine kidneys. Invest Radiol, 47, 299-305.
Haque, M., Franklin, T. & Prasad, P. 2011. Renal oxygenation changes during water loading as evaluated by BOLD MRI: effect of NOS inhibition. J Magn Reson Imaging, 33, 898-901.
Heyman, S. N., Evans, R. G., Rosen, S. & Rosenberger, C. 2012. Cellular adaptive changes in AKI: mitigating renal hypoxic injury. Nephrol Dial Transplant, 27, 1721-8.
Heyman, S. N., Rosen, S., Khamaisi, M., Idee, J. M. & Rosenberger, C. 2010. Reactive oxygen species and the pathogenesis of radiocontrast-induced nephropathy. Invest Radiol, 45, 188-95. Heyman, S. N., Rosen, S. & Rosenberger, C. 2008. Renal parenchymal hypoxia, hypoxia adaptation, and the pathogenesis of radiocontrast nephropathy. Clin J Am Soc Nephrol, 3, 288-96.
Hoff, U., Lukitsch, I., Chaykovska, L., Ladwig, M., Arnold, C., Manthati, V. L., Fuller, T. F., Schneider, W., Gollasch, M., Muller, D. N., Flemming, B., Seeliger, E., Luft, F. C., Falck, J. R., Dragun, D. & Schunck, W. H. 2011. Inhibition of 20-HETE synthesis and action protects the kidney from ischemia/reperfusion injury. Kidney Int, 79, 57-65.
Hussein, A. A., El-Dken, Z. H., Barakat, N. & Abol-Enein, H. 2012. Renal ischaemia/reperfusion injury: possible role of aquaporins. Acta Physiol (Oxf), 204, 308-16.
Jensen, A. M., Norregaard, R., Topcu, S. O., Frokiaer, J. & Pedersen, M. 2009. Oxygen tension correlates with regional blood flow in obstructed rat kidney. J Exp Biol, 212, 3156-63.
Jonsson, V., Bock, J. E. & Nielsen, J. B. 1992. Significance of plasma skimming and plasma volume expansion. J ApplPhysiol, 72, 2047-51.
Juillard, L., Lerman, L. O., Kruger, D. G., Haas, J. A., Rucker, B. C., Polzin, J. A., Riederer, S. J. & Romero, J. C. 2004. Blood oxygen level-dependent measurement of acute intra-renal ischemia. Kidney Int, 65, 944-50.
Just, A. 2007. Mechanisms of renal blood flow autoregulation: dynamics and contributions. Am J Physiol Regul Integr Comp Physiol, 292, R1-17.
Just, A., Ehmke, H., Toktomambetova, L. & Kirchheim, H. R. 2001. Dynamic characteristics and underlying mechanisms of renal blood flow autoregulation in the conscious dog. Am J Physiol Renal Physiol, 280, F1062-71.
Just, A., Ehmke, H., Wittmann, U. & Kirchheim, H. R. 2002. Role of angiotensin II in dynamic renal blood flow autoregulation of the conscious dog. J Physiol, 538, 167-77.
Le Dorze, M., Legrand, M., Payen, D. & Ince, C. 2009. The role of the microcirculation in acute kidney injury. Curr Opin Crit Care, 15, 503-8.
Legrand, M., Mik, E. G., Johannes, T., Payen, D. & Ince, C. 2008. Renal hypoxia and dysoxia after reperfusion of the ischemic kidney. Mol Med, 14, 502-16.
Leichtweiss, H. P., Lubbers, D. W., Weiss, C., Baumgartl, H. & Reschke, W. 1969. The oxygen supply of the rat kidney.: measurements of intrarenal pO1. Pflugers Arch, 309, 328-349.
Leong, C. L., Anderson, W. P., O'Connor, P. M. & Evans, R. G. 2007. Evidence that renal arterial-venous oxygen shunting contributes to dynamic regulation of renal oxygenation. Am J Physiol Renal Physiol, 292, F1726-33.
Leong, C. L., O'Connor, P. M., Eppel, G. A., Anderson, W. P. & Evans, R. G. 2008. Measurement of renal tissue oxygen tension: systematic differences between fluorescence optode and microelectrode recordings in anaesthetized rabbits. Nephron Physiol, 108, p11-7.
Li, L., Storey, P., Kim, D., Li, W. & Prasad, P. 2003. Kidneys in hypertensive rats show reduced response to nitric oxide synthase inhibition as evaluated by BOLD MRI. J Magn Reson Imaging, 17, 671-5. Li, L. P., Halter, S. & Prasad, P. V. 2008. Blood oxygen level-dependent MR imaging of the kidneys. Magn Reson Imaging Clin N Am, 16, 613-25, viii.
Li, L. P., Ji, L., Santos, E. A., Dunkle, E., Pierchala, L. & Prasad, P. 2009. Effect of nitric oxide synthase inhibition on intrarenal oxygenation as evaluated by blood oxygenation level-dependent magnetic resonance imaging. Invest Radiol, 44, 67-73.
Li, L. P., Li, B. S., Storey, P., Fogelson, L., Li, W. & Prasad, P. 2005. Effect of free radical scavenger (tempol) on intrarenal oxygenation in hypertensive rats as evaluated by BOLD MRI. J Magn Reson Imaging, 21, 245-8.
Li, L. P., Vu, A. T., Li, B. S., Dunkle, E. & Prasad, P. V. 2004. Evaluation of intrarenal oxygenation by BOLD MRI at 3.0 T. J Magn Reson Imaging, 20, 901-4.
Liss, P., Hansell, P., Carlsson, P. O., Fasching, A. & Palm, F. 2009. lodinated contrast media decrease renomedullary blood flow. A possible cause of contrast media-induced nephropathy. Adv Exp Med Biol, 645, 213-8.
Liu, Y. P., Song, R., Liang, C., Chen, X. & Liu, B. 2012. Arterial spin labeling blood flow magnetic resonance imaging for evaluation of renal injury. Am J Physiol Renal Physiol, 303, F551-8.
Lubbers, D. W. & Baumgartl, H. 1997. Heterogeneities and profiles of oxygen pressure in brain and kidney as examples of the pO2 distribution in the living tissue. Kidney Int, 51, 372-80.
Ludemann, L., Nafz, B., Elsner, F., Grosse-Siestrup, C., Meissler, M., Kaufels, N., Rehbein, H., Persson, P. B., Michaely, H. J., Lengsfeld, P., Voth, M. & Gutberlet, M. 2009. Absolute quantification of regional renal blood flow in swine by dynamic contrast- enhanced magnetic resonance imaging using a blood pool contrast agent. Invest Radiol, 44, 125-34.
Maggi, C. A., Santicioli, P. & Meli, A. 1986. Somatovesical and vesicovesical excitatory reflexes in urethane-anaesthetized rats. Brain Res, 380, 83-93.
Mathys, C., Blondin, D., Wittsack, H. J., Miese, F. R., Rybacki, K., Walther, C., Holstein, A. & Lanzman, R. S. 2011. T2' Imaging of Native Kidneys and Renal Allografts - a Feasibility Study. Rofo, 183, 112-9.
Moffat, D. B. & Fourman, J. 1963. The Vascular Pattern of the Rat Kidney. J Anat, 97, 543- 53. Navar, L. G., Inscho, E. W., Majid, S. A., Imig, J. D., Harrison-Bernard, L. M. & Mitchell, K. D. 1996. Paracrine regulation of the renal microcirculation. Physiol Rev, 76, 425-536.
Notohamiprodjo, M., Reiser, M. F. & Sourbron, S. P. 2010. Diffusion and perfusion of the kidney. Eur J Radiol, 76, 337-47.
O'Connor, P. M. 2006. Renal oxygen delivery: matching delivery to metabolic demand. Clin Exp Pharmacol Physiol, 33, 961-7.
O'Connor, P. M., Anderson, W. P., Kett, M. M. & Evans, R. G. 2006a. Renal preglomerular arterial-venous O2 shunting is a structural anti-oxidant defence mechanism of the renal cortex. Clin Exp Pharmacol Physiol, 33, 637-41.
O'Connor, P. M., Kett, M. M., Anderson, W. P. & Evans, R. G. 2006b. Renal medullary tissue oxygenation is dependent on both cortical and medullary blood flow. Am J Physiol Renal Physiol, 290, F688-94.
Ogawa, S., Lee, T. M., Kay, A. R. & Tank, D. W. 1990. Brain magnetic resonance imaging with contrast dependent on blood oxygenation. Proc Natl Acad Sci U SA, 87, 9868- 72.
Olof, P., Hellberg, A., Kallskog, O. & Wolgast, M. 1991. Red cell trapping and postischemic renal blood flow. Differences between the cortex, outer and inner medulla. Kidney Int, 40, 625-31.
Oostendorp, M., de Vries, E. E., Slenter, J. M., Peutz-Kootstra, C. J., Snoeijs, M. G., Post, M. J., van Heurn, L. W. & Backes, W. H. 2011. MRI of renal oxygenation and function after normothermic ischemia-reperfusion injury. NMR Biomed, 24, 194-200.
Pappenheimer, J. R. & Kinter, W. B. 1956. Hematocrit ratio of blood within mammalian kidney and its significance for renal hemodynamics. Am J Physiol, 185, 377-90.
Pedersen, M., Dissing, T. H., Morkenborg, J., Stodkilde-Jorgensen, H., Hansen, L. H., Pedersen, L. B., Grenier, N. & Frokiaer, J. 2005. Validation of quantitative BOLD MRI measurements in kidney: application to unilateral ureteral obstruction. Kidney Int, 67, 2305-12.
Pitts, R. F. 1974. Physilogy of the kidney and body fluids, Year Book Medical Publisher, Chicago. Prasad, P. V. 2006. Functional MRI of the kidney: tools for translational studies of pathophysiology of renal disease. Am J Physiol Renal Physiol, 290, F958-74.
Prasad, P. V. & Epstein, F. H. 1999. Changes in renal medullary p02 during water diuresis as evaluated by blood oxygenation level-dependent magnetic resonance imaging: effects of aging and cyclooxygenase inhibition. Kidney Int, 55, 294-8.
Prasad, P. V., Priatna, A., Spokes, K. & Epstein, F. H. 2001. Changes in intrarenal oxygenation as evaluated by BOLD MRI in a rat kidney model for radiocontrast nephropathy. J Magn Reson Imaging, 13, 744-7.
Regner, K. R. & Roman, R. J. 2012. Role of medullary blood flow in the pathogenesis of renal ischemia-reperfusion injury. Curr Opin Nephrol Hypertens, 21, 33-8.
Ries, M., Basseau, F., Tyndal, B., Jones, R., Deminiere, C., Catargi, B., Combe, C., Moonen, C. W. & Grenier, N. 2003. Renal diffusion and BOLD MRI in experimental diabetic nephropathy. Blood oxygen level-dependent. J Magn Reson Imaging, 17, 104-13.
Rognant, N., Guebre-Egziabher, F., Bacchetta, J., Janier, M., Hiba, B., Langlois, J. B., Gadet, R., Laville, M. & Juillard, L. 2011. Evolution of renal oxygen content measured by BOLD MRI downstream a chronic renal artery stenosis. Nephrol Dial Transplant, 26, 1205-10.
Rosen, S., Epstein, F. H. & Brezis, M. 1992. Determinants of intrarenal oxygenation: factors in acute renal failure. Ren Fail, 14, 321-5.
Schurek, H. J. 1988. [Kidney medullary hypoxia: a key to understanding acute renal failure?]. Klin Wochenschr, 66, 828-35.
Schurek, H. J., Jost, U., Baumgartl, H., Bertram, H. & Heckmann, U. 1990. Evidence for a preglomerular oxygen diffusion shunt in rat renal cortex. Am J Physiol, 259, F910-5.
Seeliger, E., Flemming, B., Wronski, T., Ladwig, M., Arakelyan, K., Godes, M., Mockel, M. & Persson, P. B. 2007. Viscosity of contrast media perturbs renal hemodynamics. J Am Soc Nephrol, 18, 2912-20.
Seeliger, E., Sendeski, M., Rihal, C. S. & Persson, P. B. 2012. Contrast-induced kidney injury: mechanisms, risk factors, and prevention. Eur Heart J, 33, 2007-15.
Seeliger, E., Wronski, T., Ladwig, M., Dobrowolski, L., Vogel, T., Godes, M., Persson, P. B. & Flemming, B. 2009. The renin-angiotensin system and the third mechanism of renal blood flow autoregulation. Am J Physiol Renal Physiol, 296, F1334-45.
Sharfuddin, A. A. & Molitoris, B. A. 2011. Pathophysiology of ischemic acute kidney injury. Nat Rev Nephrol, 7, 189-200. Silvennoinen, M. J., Clingman, C. S., Golay, X., Kauppinen, R. A. & van Zijl, P. C. 2003. Comparison of the dependence of blood R2 and R2* on oxygen saturation at 1.5 and 4.7 Tesla. Magn Reson Med, 49, 47-60.
Singh, P., Gupta, M. & Srivastava, A. 2008. Nitrous oxide during anesthesia for laparoscopic donor nephrectomy: Does it matter? Indian J Urol, 24, 126-7.
Srisawat, N. & Kellum, J. A. 2011. Acute kidney injury: definition, epidemiology, and outcome. Curr Opin Crit Care, 17, 548-55.
Tumlin, J. A. 2009. Impaired blood flow in acute kidney injury: pathophysiology and potential efficacy of intrarenal vasodilator therapy. Curr Opin Crit Care, 15, 514-9.
Turner, R., Jezzard, P., Wen, H., Kwong, K. K., Le Bihan, D., Zeffiro, T. & Balaban, R. S. 1993. Functional mapping of the human visual cortex at 4 and 1.5 tesla using deoxygenation contrast EPI. Magn Reson Med, 29, 277-9.
Twombley, K., Baum, M. & Gattineni, J. 2011. Accidental and iatrogenic causes of acute kidney injury. Curr Opin Pediatr, 23, 208-14.
van der Zwaag, W., Francis, S., Head, K., Peters, A., Gowland, P., Morris, P. & Bowtell, R. 2009. fMRI at 1.5, 3 and 7 T: characterising BOLD signal changes. Neuroimage, 47, 1425-34.
Wang, J., Zhang, Y., Yang, X., Wang, X., Zhang, J., Fang, J. & Jiang, X. 2012. Hemodynamic effects of furosemide on renal perfusion as evaluated by ASL-MRI. Acad Radiol, 19, 1194-200. Warner, L., Glockner, J. F., Woollard, J., Textor, S. C., Romero, J. C. & Lerman, L. O. 2011. Determinations of renal cortical and medullary oxygenation using blood oxygen level- dependent magnetic resonance imaging and selective diuretics. Invest Radiol, 46, 41- 7.
Warner, L., Gomez, S. I., Bolterman, R., Haas, J. A., Bentley, M. D., Lerman, L. O. & Romero, J. C. 2009. Regional decreases in renal oxygenation during graded acute renal arterial stenosis: a case for renal ischemia. Am J Physiol Regul Integr Comp Physiol, 296, R67-71.
Welch, W. J., Baumgartl, H., Lubbers, D. & Wilcox, C. S. 2001. Nephron pO2 and renal oxygen usage in the hypertensive rat kidney. Kidney Int, 59, 230-7.
Wronski, T., Seeliger, E., Persson, P. B., Forner, C., Fichtner, C., Scheller, J. & Flemming, B. 2003. The step response: a method to characterize mechanisms of renal blood flow autoregulation. Am J Physiol Renal Physiol, 285, F758-64.
Yap, S. C. & Lee, H. T. 2012. Acute kidney injury and extrarenal organ dysfunction: new concepts and experimental evidence. Anesthesiology, 116, 1139-48.
Zarjou, A. & Agarwal, A. 2011. Sepsis and acute kidney injury. J Am Soc Nephrol, 22, 999- 1006. Zarjou, A., Sanders, P. W., Mehta, R. L. & Agarwal, A. 2012. Enabling innovative translational research in acute kidney injury. Clin Transl Sci, 5, 93-101.
Zhang, Y., Wang, J., Yang, X., Wang, X., Zhang, J., Fang, J. & Jiang, X. 2012. The serial effect of iodinated contrast media on renal hemodynamics and oxygenation as evaluated by ASL and BOLD MRI. Contrast Media Mol Imaging, 7, 418-25.
Zhao, J. M., Clingman, C. S., Narvainen, M. J., Kauppinen, R. A. & van Zijl, P. C. 2007. Oxygenation and hematocrit dependence of transverse relaxation rates of blood at 3T. Magn Reson Med, 58, 592-7.
Zimmerhackl, B., Dussel, R. & Steinhausen, M. 1985. Erythrocyte flow and dynamic hematocrit in the renal papilla of the rat. Am J Physiol, 249, F898-902.

## Claims

1. System for simultaneous determination of renal perfusion and renal oxygenation via in vivo measurement and magnetic resonance imaging (MRI) comprising
- a perivascular flow probe for measuring renal blood flow,
- two oxygen probes for measuring cortical and medullary oxygen saturation, in particular partial pressure of oxygen (pO2), respectively,
- an animal holding device, and
- an MRI-scanner, preferably a small-bore animal MRI scanner,
wherein the animal holding device
- is of a half-pipe shape,
- comprises a radio frequency surface coil array for MRI signal reception,
- comprises a bridge structure for securing the leads for physiological probes, wherein the bridge structure is adjustable in height and distance to the animal and
- is of suitable dimensions for fitting, after animal and probe mounting, within a small-bore animal MRI scanner, which comprises a magnet bore with a clearance of a diameter of 15 to 40 cm, preferably 20 cm.

2. System according to the preceding claim comprising one or more near infrared probes suitable for detection or measurement of oxygen saturation and/or haemoglobin concentration.

3. System according to any one of the preceding claims, **characterized in that** the perivascular flow probe comprises an acoustic reflector of ceramic material, preferably glass-ceramic.

4. System according to any one of the preceding claims, **characterized in that** the perivascular flow probe comprises wing-shaped
and/or silicone-reinforced gauze for fixation to tissue, and/or comprises no vessel locking device.

5. System according to any one of the preceding claims, **characterized in that** the oxygen probes are laser-Doppler-flux and pO2 probes, preferably between 0.1 and 2 mm in diameter, preferably 0.5 mm.

6. System according to any one of the preceding claims, **characterized in that** the oxygen probe comprises fibres, wherein said fibers are encased by silicone tubing, which is fitted with a silicone cap, preferably of silicone tape.

7. System according to any one of the preceding claims, **characterized in that** the distance between the silicone cap of the oxygen probes and probe tip within the silicone tubing is 0.2 to 3 mm, preferably 0.5 to 2 mm, more preferably 1 to 1.5 mm for a cortical probe, or 1 to 6 mm, preferably 2 to 5 mm, more preferably 3 to 4 mm for a medullary probe, respectively.

8. System according to any one of the preceding claims comprising one or more catheters, for example for the femoral artery and/or femoral vein,
and/or comprising an inflatable aortic cuff, preferably of silicone and/or polyethylene material.

9. System according to any one of the preceding claims comprising means to support standardized interventions in the MR scanner including hypoxia/hyperoxia or other chemically, biologically or pharmacologically induced interventions, wherein said means are selected from catheters, infusion or intravenous apparatus for providing a drug or other treatment.

10. System according to any one of the preceding claims, **characterized in that** the MRI-scanner comprises a magnet bore with a clearance of a diameter of 15 to 30 cm, preferably 20 cm.

11. System according to any one of the preceding claims, **characterized in that** the MRI-scanner has greater than 7 T, preferably a 9.4 T scanner.

12. System according to any one of the preceding claims, **characterized in that** the MRI-scanner is configured to carry out or comprises a T2*/T2 weighted MRI, water diffusion MRI, perfusion MR, T1 weighted MRI, renal quantitative susceptibility mapping, kidney size or MR based oxygenation and/or glomerular function analysis.

13. System according to any one of the preceding claims, **characterized in that** the MRI-scanner is configured to detail the link between renal tissue pO2, renal blood flow, kidney size, conductance, flux and T2*/T2 weighted MRI, water diffusion MRI, perfusion MR, T1 weighted MRI, renal quantitative susceptibility, kidney size or MR based oxygenation and/or glomerular function analysis.

## Patentansprüche

1. System zur gleichzeitigen Bestimmung von Nierenperfusion und renaler Sauerstoffversorgung über In-vivo-Messung und Magnetresonanztomographie (MRT), das Folgendes umfasst:
- eine perivaskuläre Strömungssonde zum Messen der renalen Blutströmung,
- zwei Sauerstoffsonden zum Messen der kortikalen bzw. medullären Sauerstoffsättigung, insbesondere des SauerstoffPartialdrucks (pO2),
- eine Tierhaltevorrichtung, und
- einen MRT-Scanner, vorzugsweise ein Tier-MRT-Scanner mit kleiner Bohrung,
wobei die Tierhaltevorrichtung
- eine Halbröhren-Form aufweist,
- eine Funkfrequenz-Oberflächenspulenanordnung zum Empfangen von MRT-Signalen umfasst,
- eine Brückenstruktur zum Sichern der Leitungen für physiologische Sonden umfasst, wobei die Brückenstruktur in Höhe und Abstand zu dem Tier einstellbar ist, und
- geeignete Abmessungen aufweist, um, nachdem das Tier und die Sonden in Stellung gebracht wurden, in einen Tier-MRT-Scanner mit kleiner Bohrung hineinzupassen, der eine Magnetbohrung mit einem Freiraum umfasst, der einen Durchmesser von 15 bis 40 cm, vorzugsweise von 20 cm, aufweist.

2. System nach dem vorhergehenden Anspruch, das eine oder mehrere Nahinfrarotsonden umfasst, die zum Erfassen oder Messen von Sauerstoffsättigung und/oder Hämoglobinkonzentration geeignet sind.

3. System nach einem der vorhergehenden Ansprüche, **dadurch gekennzeichnet, dass** die perivaskuläre Strömungssonde einen akustischen Reflektor aus keramischem Material, vorzugsweise Glaskeramik, umfasst.

4. System nach einem der vorhergehenden Ansprüche, **dadurch gekennzeichnet, dass** die perivaskuläre Strömungssonde flügelförmige und/oder silikonverstärkte Gaze zur Fixierung an Gewebe umfasst und/oder keine Gefäßverschlussvorrichtung umfasst.

5. System nach einem der vorhergehenden Ansprüche, **dadurch gekennzeichnet, dass** die Sauerstoffsonden Laser-Doppler-Flux- und pO2-Sonden sind, vorzugsweise mit einem Durchmesser zwischen 0,1 und 2 mm, vorzugsweise 0,5 mm.

6. System nach einem der vorhergehenden Ansprüche, **dadurch gekennzeichnet, dass** die Sauerstoffsonde Fasern umfasst, wobei die Fasern durch einen Silikonschlauch umhüllt sind, der mit einer Silikonkappe versehen ist, die vorzugsweise aus Silikonband besteht.

7. System nach einem der vorhergehenden Ansprüche, **dadurch gekennzeichnet, dass** der Abstand zwischen der Silikonkappe der Sauerstoffsonden und der Sondenspitze innerhalb des Silikonschlauches 0,2 bis 3 mm, vorzugsweise 0,5 bis 2 mm, besonders bevorzugt 1 bis 1,5 mm für eine kortikale Sonde, bzw. 1 bis 6 mm, vorzugsweise 2 bis 5 mm, besonders bevorzugt 3 bis 4 mm für eine medulläre Sonde beträgt.

8. System nach einem der vorhergehenden Ansprüche, das einen oder mehrere Katheter umfasst, beispielsweise für die femorale Arterie und/oder die femorale Vene,
und/oder eine aufblasbare Aorten-Manschette umfasst, die vorzugsweise aus Silikon- und/oder Polyethylenmaterial besteht.

9. System nach einem der vorhergehenden Ansprüche, das Mittel umfasst, um standardisierte Eingriffe in dem MR-Scanner zu unterstützen, einschließlich Hypoxie/Hyperoxie oder andere chemisch, biologisch oder pharmakologisch induzierte Eingriffe, wobei die Mittel aus Kathetern, einer Infusion oder einem intravenösen Gerät zum Bereitstellen eines Medikaments oder einer anderen Behandlung ausgewählt sind.

10. System nach einem der vorhergehenden Ansprüche, **dadurch gekennzeichnet, dass** der MRT-Scanner eine Magnetbohrung mit einem Freiraum umfasst, der einen Durchmesser von 15 bis 30 cm, vorzugsweise 20 cm, aufweist.

11. System nach einem der vorhergehenden Ansprüche, **dadurch gekennzeichnet, dass** der MRT-Scanner einen Scanner mit mehr als 7 T, vorzugsweise einen Scanner mit 9,4 T aufweist.

12. System nach einem der vorhergehenden Ansprüche, **dadurch gekennzeichnet, dass** der MRT-Scanner dazu konfiguriert ist, eine T2*/T2-gewichtete MRT, eine Wasserdiffusions-MRT, eine Perfusion-MR, eine T1-qéwlchtété MRT, renale quantitative Suszeptibilitätskartierung, nierengrößen- oder MR-basierte Analyse der Sauerstoffversorgung und/oder der glomerulären Funktion durchzuführen oder diese umfasst.

13. System nach einem der vorhergehenden Ansprüche, **dadurch gekennzeichnet, dass** der MRT-Scanner dazu konfiguriert ist, die Verbindung zwischen pO2 des renalen Gewebes, renalem Blutfluss, Nierengröße, Leitfähigkeit, Fluss und T2*/T2-gewichteter MRT, Wasserdiffusions-MRT, Perfusion-MR, T1-qéwlchtétér MRT, renaler quantitativer Suszeptibilität, nierengrößen- oder MR-basierter Analyse der Sauerstoffversorgung und/oder der glomerulären Funktion genau zu beschreiben.

## Revendications

1. Système pour la détermination simultanée d'une perfusion rénale et d'une oxygénation rénale via une mesure in vivo et une imagerie par résonance magnétique (IRM) comprenant
- une sonde de débit périvasculaire pour mesurer le débit sanguin rénal,
- deux sondes à oxygène pour mesurer la saturation en oxygène corticale et médullaire, en particulier la pression partielle d'oxygène (pO2), respectivement,
- un dispositif de maintien d'animal, et
- un scanner IRM, de préférence un scanner IRM d'animal de petite taille,
dans lequel le dispositif de maintien d'animal
- a une forme en demi-lune,
- comprend un groupement de bobines de surface de radiofréquence pour la réception de signaux IRM,
- comprend une structure de pont pour fixer les câbles pour les sondes physiologiques, dans lequel la structure de pont est réglable en hauteur et en distance par rapport à l'animal et
- est de dimensions appropriées pour rentrer, après l'installation de l'animal et de la sonde, dans un scanner IRM d'animal de petite taille, qui comprend un alésage d'aimant avec un espace d'un diamètre de 15 à 40 cm, de préférence 20 cm.

2. Système selon la revendication précédente comprenant une ou plusieurs sondes infrarouges proches appropriées pour la détection ou la mesure de la saturation en oxygène et/ou de la concentration en hémoglobine.

3. Système selon l'une quelconque des revendications précédentes, **caractérisé en ce que** la sonde de débit périvasculaire comprend un réflecteur acoustique en matériau céramique, de préférence vitrocéramique.

4. Système selon l'une quelconque des revendications précédentes, **caractérisé en ce que** la sonde de débit périvasculaire comprend une gaze en forme d'aile et/ou renforcée au silicone pour la fixation aux tissus, et/ou ne comprend aucun dispositif de verrouillage de cuve.

5. Système selon l'une quelconque des revendications précédentes, **caractérisé en ce que** les sondes à oxygène sont des sondes laser-Doppler-flux et pO2, de préférence entre 0,1 et 2 mm de diamètre, de préférence 0,5 mm.

6. Système selon l'une quelconque des revendications précédentes, **caractérisé en ce que** la sonde à oxygène comprend des fibres, dans lequel lesdites fibres sont enrobées par un tube de silicone, qui est équipé d'un bouchon de silicone, de préférence d'une bande de silicone.

7. Système selon l'une quelconque des revendications précédentes, **caractérisé en ce que** la distance entre le capuchon de silicone des sondes à oxygène et l'embout de sonde à l'intérieur du tube de silicone est de 0,2 à 3 mm, de préférence de 0,5 à 2 mm, plus préférablement de 1 à 1,5 mm pour une sonde corticale, ou de 1 à 6 mm, de préférence de 2 à 5 mm, plus préférablement de 3 à 4 mm pour une sonde médullaire, respectivement.

8. Système selon l'une quelconque des revendications précédentes, comprenant un ou plusieurs cathéters, par exemple pour l'artère fémorale et/ou la veine fémorale,
et/ou comprenant un manchon aortique gonflable, de préférence d'un matériau de silicone et/ou de polyéthylène.

9. Système selon l'une quelconque des revendications précédentes, comprenant des moyens pour prendre en charge des interventions normalisées dans le scanner RM, y compris des inventions hypoxie/hyperoxie ou d'autres interventions induites chimiquement, biologiquement ou pharmacologiquement, dans lequel lesdits moyens sont choisis parmi les cathéters, une perfusion ou un appareil intraveineux pour délivrer un médicament ou un autre traitement.

10. Système selon l'une quelconque des revendications précédentes, **caractérisé en ce que** le scanner IRM comprend un alésage d'aimant avec un espace d'un diamètre de 15 à 30 cm, de préférence 20 cm.

11. Système selon l'une quelconque des revendications précédentes, **caractérisé en ce que** le scanner IRM a un scanner supérieur à 7 T, de préférence un scanner de 9,4 T.

12. Système selon l'une quelconque des revendications précédentes, **caractérisé en ce que** le scanner IRM est configuré pour effectuer ou comprend une IRM pondérée T2*/T2, une IRM à diffusion d'eau, une RM de perfusion, une IRM pondérée T1, une cartographie de susceptibilité quantitative rénale, la taille du rein ou une oxygénation basée sur la RM et/ou une analyse de la fonction glomérulaire.

13. Système selon l'une quelconque des revendications précédentes, **caractérisé en ce que** le scanner IRM est configuré pour détailler le lien entre la pO2 des tissus rénaux, le débit sanguin rénal, la taille des reins, la conductance, le flux et une IRM pondérée T2*/T2, une IRM à diffusion d'eau, une RM de perfusion, une IRM pondérée T1, une susceptibilité quantitative rénale, la taille du rein ou une oxygénation basée sur la RM et/ou une analyse de la fonction glomérulaire.
